(19) Europäisches Patentamt
European Patent Office
Office européen des brevets

(11) **EP 4 609 916 A2**

(12) **EUROPEAN PATENT APPLICATION**

(43) Date of publication:
**03.09.2025** Bulletin 2025/36

(21) Application number: **25174128.6**

(22) Date of filing: **01.05.2020**

(51) International Patent Classification (IPC):
***A61P 37/06*** (2006.01)

(52) Cooperative Patent Classification (CPC):
**C07K 16/2803; A61P 37/06;** C07K 2317/34;
C07K 2317/75; C07K 2317/92

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **01.05.2019 GB 201906118**

(62) Document number(s) of the earlier application(s) in
accordance with Art. 76 EPC:
**20725994.6 / 3 962 526**

(71) Applicant: **Immutep S.A.S.**
**91190 Saint-Aubin (FR)**

(72) Inventors:
• **TRIEBEL, Frédéric**
**91190 SAINT-AUBIN (FR)**
• **BRIGNONE, Chrystelle**
**91190 SAINT-AUBIN (FR)**

(74) Representative: **Reddie & Grose LLP**
**The White Chapel Building**
**10 Whitechapel High Street**
**London E1 8QS (GB)**

Remarks:
•The complete document including Reference
Table(s) and the Sequence Listing(s) can be
downloaded from the EPO website
•This application was filed on 03-05-2025 as a
divisional application to the application mentioned
under INID code 62.
•Claims filed after the date of filing of the application /
after the date of receipt of the divisional application
(Rule 68(4) EPC).

(54) **ANTI-LAG-3 BINDING MOLECULES**

(57)     Binding molecules that bind specifically to Lymphocyte-activation gene-3 (LAG-3) are described. The binding molecules inhibit T cell receptor (TCR)-mediated signal transduction in LAG-3 positive T cells through agonism of LAG-3. In some embodiments, the binding molecules bind specifically to a discontinuous epitope within the extracellular Ig superfamily domain D1 of a LAG-3 protein, wherein amino acid residues of the discontinuous epitope lie outside a 30 amino acid extra-loop sequence of the domain D1 of the LAG-3 protein. Use of the binding molecules as medicaments, in particular for the treatment of conditions associated with proliferation and/or activation of CD4$^+$ and/or CD8$^+$ T cells, in particular inflammatory and autoimmune disorders, is also described.

**Description**

This invention relates to binding molecules, such as antibodies, or antigen-binding fragments thereof, that bind specifically to Lymphocyte-activation gene-3 (LAG-3), and to use of the binding molecules as medicaments, in particular for the treatment of conditions associated with proliferation and/or activation of CD4$^+$ and/or CD8$^+$ T cells, in particular inflammatory and autoimmune disorders.

Stimulation of T cell function is initiated upon interaction of the T cell receptor (TCR) with short peptides presented by MHC class I or II molecules (MHC I for CD8 T cells, MHC II for CD4 T cells) on the surface of antigen-presenting cells (APCs). The TCR by itself is not capable of activating downstream pathways to initiate T cell activation. This also requires co-receptors, such as CD4 for helper T cells, and CD8 for cytotoxic T cells. These co-receptors bind their respective MHC molecules and stabilise the interaction of T cells and APCs. In addition to TCR binding to antigen-loaded MHC, both helper T cells and cytotoxic T cells require a number of secondary signals to become activated and respond. In the case of helper T cells, the first of these is provided by CD28. This molecule on the T cell binds to one of two molecules on the APC (CD80 or CD86) and initiates T-cell proliferation, leading to the expansion of T cell clones specific for the antigen. Cytotoxic T cells are less reliant on CD28 for activation, but do require signals from other co-stimulatory molecules such as CD70 and CD137.

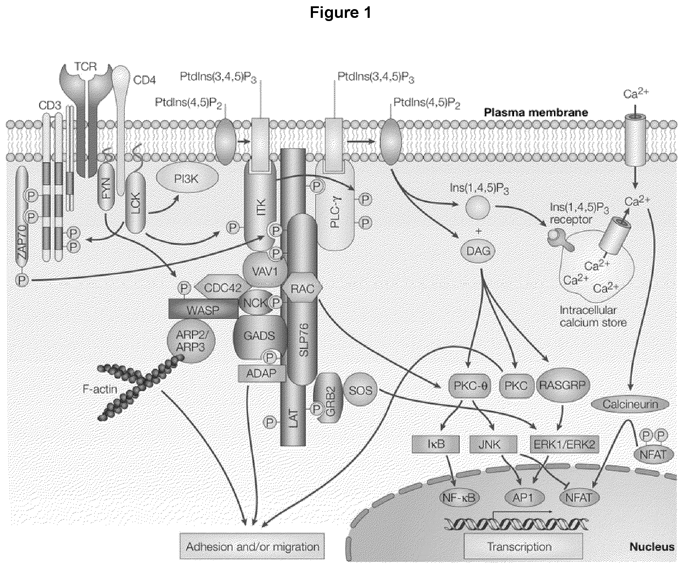

The TCR is located in close proximity to a complex of signaling molecules, which mediate T cell activation through multiple signaling cascades (see Figure 1 for an overview of TCR signaling). These signaling molecules include the CD3 family of proteins. Once the TCR is properly engaged with the peptide-MHC complex, conformational changes in the associated CD3 chains are induced, which leads to their phosphorylation and association with downstream proteins. The TCR zeta chain is also phosphorylated upon TCR engagement. These molecules are phosphorylated by the Src kinases, leukocyte-specific tyrosine kinase (LCK) and Fyn, via their C-terminal Immunoreceptor Tyrosine-based Activation Motifs (ITAMs).

Phosphorylated CD3 ITAMs recruit and activate the Syk family kinase zeta-activated protein 70 kDa (ZAP70). ZAP70 then phosphorylates a membrane associated scaffolding protein called Linker for Activation of T cells (LAT). LAT in turn recruits a second molecular scaffold, SH2-domain-containing Leukocyte Protein of 76 kDa (Slp-76). Slp-76 is then phosphorylated by ZAP70 and the resulting LAT-Slp-76 complex acts as a scaffold for the recruitment of signalling effector molecules. Interleukin-2 inducible tyrosine kinase (ITK) then interacts with the LAT-Slp-76 complex and becomes activated by auto-phosphorylation. This promotes phosphorylation of the effector molecule phospholipase C gamma (PLC-y1). PLC-y1 transduces TCR signals by cleaving phosphatidylinositol triphosphate (PIP$_2$) in the plasma membrane to generate the second messengers diacylglycerol (DAG) and inositol trisphosphate (IP$_3$).

DAG, a membrane associated lipid, activates a number of downstream proteins, including various isoforms of protein kinase C (PKC) and RAS guanyl nucleotide-releasing protein (RasGRP). Upon activation by DAG, PKC-theta participates in activating the NF-κB pathway, whereas RasGRP is a crucial activator of MAPK signalling pathways. IP$_3$ stimulates the efflux of Ca$^{2+}$ from the endoplasmic reticulum to the cytoplasm. Elevated Ca$^{2+}$ levels induce activation of the protein phosphatase, calcineurin, which then dephosphorylates the T cell transcription factor nuclear factor of activated T cells (NFAT). Dephosphorylated NFAT then migrates to the nucleus to join other transcription factors in inducing the transcription of specific genes. NFAT differentially regulates the expression of genes related to T cell development, activation, and differentiation.

Uncontrolled immune responses to pathogens or self-antigens can cause inflammatory tissue damage and autoimmune diseases. To prevent this, immune responses are regulated by a balance between co-stimulatory and inhibitory signals, collectively referred to as immune checkpoints, which are necessary for maintaining self-tolerance and protecting the host from tissue damage. Activated T cells express multiple co-inhibitory receptors, such as lymphocyte-activation gene 3 (LAG-3), programmed cell death protein 1 (PD-1), cytotoxic T-lymphocyte-associated protein 4 (CTLA-4), and T cell immunoglobulin and immunoreceptor tyrosine-based inhibitory motif [ITIM] domain (TIGIT). Inhibitory immune checkpoint receptors have been shown to modulate T cell responses to self-proteins as well as to chronic infections and tumor antigens. Inhibitory immune checkpoint receptors are targets for cancer immunotherapy due to their potential for use in multiple types of cancers.

Considering the important role of NFAT signalling in T cell function, NFAT has long been considered as an attractive target for therapeutic approaches to control autoimmune responses and graft rejection (reviewed in Lee et al., Frontiers in Immunology, 2018, Vol. 9, Article 2747 - see Table 2). Calcineurin inhibitors, such as cyclosporine A (CsA) and tacrolimus (FK506), inhibit the phosphatase activity of calcineurin, thereby inhibiting NFAT dephosphorylation. Both drugs have been used to treat graft rejection and autoimmune diseases. However, although calcineurin inhibitors are effective in autoimmune disease therapy with inhibition of T cell activation, they are associated with serious side effects. For example, blocking of calcineurin phosphatase activity affects numerous targets of calcineurin as well as NFATs, with neurotoxicity and nephrotoxicity being the most common side effects. Endothelin level is also increased, and is related to endothelial dysfunction, impaired glomerular filtration, and systemic hypertension. Calcineurin inhibitors also have a negative effect on regulatory T cell proliferation and function, which are necessary for immune tolerance.

Small molecule inhibitors are similar in structure and function to classical inhibitors but have lesser side effects.

For example, drugs have been developed that inhibit T cell responses by enhancing nuclear export of NFAT1 and NFAT2, induce NFAT1 degradation via GSK3b inhibition, or block the binding of NFAT to DNA. However, they are yet to be analysed in suitable animal models of autoimmune diseases to investigate their potential in ameliorating diseases. In particular, NFAT targeting strategies should consider non-T cell mediated adverse effects. Recent studies in myeloid cells have revealed the importance of NFAT in both innate and adaptive immunity. In an early response to pathogens, pattern recognition receptors (PRRs) induce the production of IL-2 from dendritic cells. These signals activate PLCy2 and promote NFAT-dependent IL-2 expression. In macrophages, calcineurin/NFAT inhibitor treatment results in macrophages that are tolerant to a lethal dose of lipopolysaccharide (LPS). Other myeloid cells such as mast cells and neutrophils are influenced by Ca2+/NFAT signaling and produce cytokines and multiple immune-mediators.

In view of the multiple roles of calcineurin-NFAT signaling in both immune and non-immune cells, and the adverse side effects of calcineurin inhibitors, there is a need to identify more selective and less toxic inhibitors of calcineurin-NFAT signaling in T cells.

LAG-3 is a CD4 homolog type I membrane protein with four extracellular Ig superfamily domains. Similar to CD4, LAG-3 oligomerizes at the surfaces of T cells and binds to MHC class II molecules on antigen-presenting cells (APCs) but with significantly higher affinity than CD4. LAG-3 is expressed on activated CD4$^+$ and CD8$^+$ T lymphocytes where it associates with the CD3-TCR complex at the cell surface and negatively regulates signal transduction. As a consequence, it negatively regulates T cell proliferation, function, and homeostasis. When recognition of the MHC class II-peptide complex by a specific TCR occurs, intracellular signals are transduced in the T cell through the TCR and in the APC through MHC class II molecules. The negative regulatory role of LAG-3 signalling into T cells operates in primary CD4 and CD8 human T-cell responses (Maçon-Lemaître, et al., Immunology. 2005 Jun; 115(2):170-178).

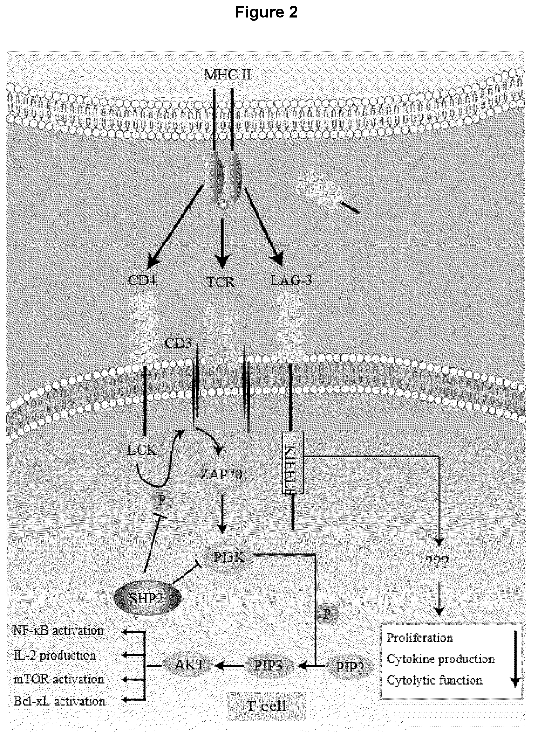

The molecular mechanism by which LAG-3 negatively regulates signal transduction in T cells is not known (see Figure 2). The inhibitory function of LAG-3 requires its intracellular (IC) region, suggesting that LAG-3 transduces inhibitory signal via its IC region. The IC region of LAG-3 comprises about 60 amino acid residues, which do not contain a typical signalling motif with a known signalling mechanism, such as an immuno-receptor tyrosine based inhibitory motif (ITIM) or an immuno-receptor tyrosine-based switch motif (ITSM). Workman et al. (J. Immunol. 2002, 169, 5392-5395) reported that a KIEELE motif in the middle of the IC region is conserved among species and is required for LAG-3 to inhibit antigen-dependent activation of 3A9 hybridoma T cells. Substitution of the K residue with A was sufficient to abrogate the inhibitory capacity of LAG-3. However, the mechanism by which this K residue transduces inhibitory signal is currently unknown. Iouzalen et al. (Eur. J. Immunol. 2001, 31, 2885-2891) reported that LAG-3 associated protein (LAP) binds to the C-terminal region of LAG-3 with 10-15 repeats of E and preferably, but not limited to, P (an EX repeat). The function of LAP and the EX repeat also remain unknown. Maeda et al. (J. Biol. Chem. 2019, RA119.007455) have recently reported that LAG-3 transduces two independent inhibitory signals through an FxxL motif in the membrane-proximal region and the C-terminal EX repeat. The authors note that these motifs have not previously been reported for inhibitory co-receptors, and suggest that LAG-3 inhibits T cell activation through a non-redundant inhibitory mechanism along with the other inhibitory co-receptors. However, the molecular mechanism of the inhibitory signal transduced by LAG-3 (i.e. the LAG-3 signalling pathway) remains elusive.

Agents capable of modulating the activation and/or effector functions of CD8-positive and CD4-positive T cells are highly desirable. In particular, many autoimmune disorders are known to involve autoreactive T cells and autoantibodies. There is a need, therefore, for agents that are capable of inhibiting or eliminating autoreactive lymphocytes without compromising the immune system's ability to defend against pathogens. Poirier et al (Clinical and Experimental Immunology, 2011, 164:265-274) describe evaluation of a cytotoxic LAG-3 chimeric antibody (chimeric A9H12). *In vivo,* the antibody depleted LAG-3$^+$-activated T cells in lymph nodes and showed efficacy at reducing skin inflammation in a tuberculin-induced delayed-type hypersensitivity (DTH) model in baboons. Antibodies that specifically deplete activated T cells present a promising therapeutic strategy to prevent and/or treat autoimmune disorders.

As an alternative strategy, the applicant has appreciated that LAG-3 agonists will negatively regulate T cell proliferation and/or function without depleting the T cells, and that such agonists can also be used to treat inflammatory and autoimmune disorders. The applicant has been able to produce the first anti-LAG-3 agonistic antibodies, which are described in WO 2017/037203. These antibodies inhibit antigen-induced proliferation of CD4-positive and CD8-positive T cells. They include mouse monoclonal antibody 13E2, and humanized 13E2-human IgG4 Fc antibody (referred to as IMP761). However, the mechanism by which IMP761, through agonism of LAG-3, negatively regulates signal transduction within T-cells is not known, neither is the LAG-3 epitope known to which IMP761 binds.

It has now surprisingly been found that IMP761, through agonism of LAG-3, inhibits TCR signalling, in particular NFAT-regulated gene expression, in LAG-3 positive T-cells. LAG-3 is expressed primarily on activated CD4$^+$ and CD8$^+$ T cells, and on natural killer (NK) cells, so this finding allows for inhibition of TCR signalling (in particular NFAT signalling) in activated T cells, through agonism of LAG-3, without directly inhibiting calcineurin, and with minimal non-T cell-mediated effects. The epitope to which IMP761 binds has also been identified.

According to the invention there is provided an isolated binding molecule, which is an agonist of LAG-3, and which inhibits TCR-mediated signal transduction in LAG-3 positive T cells through agonism of LAG-3.

Signal transduction (also known as cell signalling) is the transmission of molecular signals from the exterior of a cell to its interior.

The term "agonist of LAG-3" is used herein to refer to a molecule that is able to bind to LAG-3 on the surface of a T-cell and induce a response, in particular an inhibition of TCR-mediated signal transduction within the T cell.

Any TCR-mediated signalling pathway within the T cells may be inhibited. For example, the NF-κB pathway (in particular, a PKC-theta-dependent NF-κB pathway), the RasGRP-dependent MAPK signalling pathway (in particular a MAPK/ERK signalling pathway), or the calcineurin/NFAT signalling pathway within the T cells may be inhibited. In particular, the calcineurin/NFAT signalling pathway may be inhibited. A downregulation of calcineurin-NFAT signaling within the T cells may result in reduced expression of one or more NFAT-regulated genes.

Inhibition of expression of a NFAT-regulated gene includes a reduction in expression of the gene compared with expression of the gene in the absence of the isolated binding molecule. Inhibition of expression of a NFAT-regulated gene by a binding molecule which is an agonist of LAG-3 may be determined by any suitable method. Expression of the gene in the presence of the binding molecule may be reduced by at least 10%, at least 20%, at least 30%, at least 40%, at least 50%, at least 60%, at least 70%, at least 80%, at least 90%, or 100%, compared to expression of the gene in the absence of the binding molecule.

All NFAT proteins share a conserved core region comprising a DNA-binding REL-homology domain and a less conserved N-terminal regulatory domain (also known as a NFAT-homology domain) (reviewed in Vaeth & Feske, 2018, F1000 Faculty Rev:260). The NFAT transcription factor family consists of five members NFAT1, NFAT2, NFAT3, NFAT4, and NFAT5. NFAT1, 2, 3, and 4 are regulated by calcium signalling. NFAT5 has special characteristics that differentiate it from other NFAT members. Calcium signalling is critical to NFAT activation because calmodulin (CaM), a calcium sensor protein, activates the serine/threonine phosphatase calcineurin (CN). Activated CN rapidly dephosphorylates the serine-rich region (SRR) and SP-repeats in the amino termini of NFAT proteins, resulting in a conformational change that exposes a nuclear localization signal, resulting in NFAT nuclear import. NFAT1, 2 and 4 are known to play important roles in T cell activation. NFAT3 has not been shown to be expressed in T cells, and NFAT5 has not so far been highlighted in T cell function.

NFAT proteins have weak DNA-binding capacity on their own, so must cooperate with other nuclear resident transcription factors to effectively bind DNA, including AP1 (formed by the transcription factors c-Jun and c-Fos), FOXP3, and BATF. NFAT is involved in transcriptional regulation of numerous cytokines, chemokines, and growth factors in immune cells. Activation of NFAT and AP-1 is known to be required for productive immune responses. Cooperation of NFAT with AP-1 is required for many different genes to be transcribed, including IL-2, the IL-2 receptor, GM-CSF, IL-3, IL-4, and IFN-γ. NFAT-dependent promoters and enhancers tend to have 3-5 NFAT binding sites.

Stimulation of effector T cells via the TCR and co-stimulatory receptors results in NFAT activation and its cooperative DNA binding with AP-1. NFAT:AP-1 complexes regulate the expression of cytokines and other effector molecules as well as the short NFAT2/αA isoform that further enhances T-cell activation in a positive auto-regulatory loop (Vaeth & Feske, *supra*). Klein-Hessling et al. (Nature Communications, 2017, 8:511) have reported that NFAT1 controls the cytotoxicity of CD8[+] T cells. Transcriptome analysis shows diminished RNA levels of numerous genes in Nfatc1[-/-] CD8[+] T cells, including genes encoding cytokines and chemokines. Genome-wide ChIP-seq shows that NFAT1 binds many genes that control cytotoxic T lymphocyte activity.

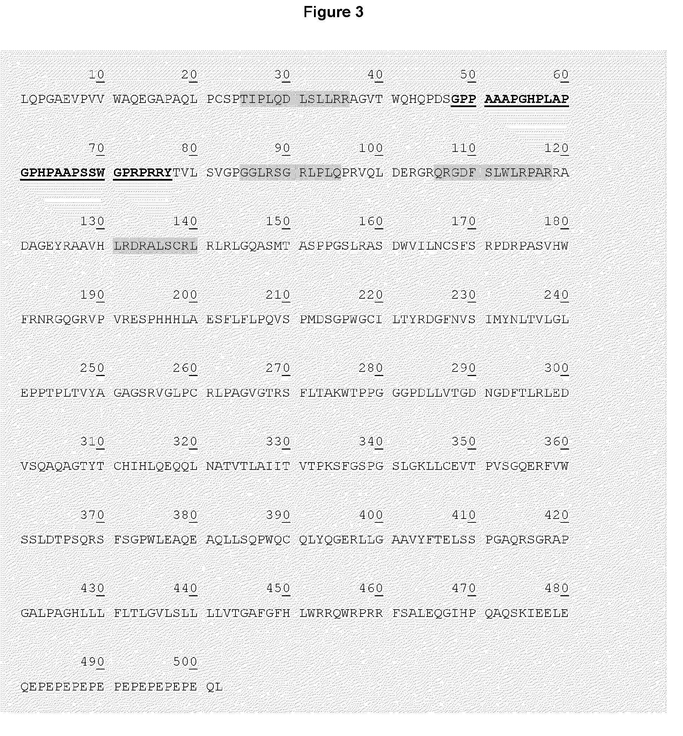

NFAT (in particular, NFAT2) acts as a central regulator of T-cell metabolism (reviewed in Vaeth & Feske, *supra*). Naïve T cells are metabolically quiescent and characterized by minimal nutrient uptake, low glycolysis, and effective oxidative phosphorylation. Activated lymphocytes reset their metabolism to a high-rate anabolic metabolism fuelled by aerobic glycolysis that supports the synthesis of macromolecules required for clonal expansion. CD4[+] T cells lacking NFAT activation failed to undergo this "glycolytic switch" and antigen receptor-induced clonal expansion. $Ca^{2+}$/calcineurin/NFAT signalling following T-cell receptor (TCR) and co-stimulation is required for the switch from catabolic to high-rate anabolic metabolism of activated T cells, cell cycle entry, and proliferation. NFAT directly controls the expression of "metabolic master regulators" such as IRF4, HIF1α, and potentially c-Myc that subsequently induce the expression of glucose transporter 1 (GLUT1), glycolytic enzymes, and mitochondrial electron transport chain (ETC) complexes that mediate aerobic glycolysis and mitochondrial respiration, respectively. In addition, NFAT directly controls the expression of the high-affinity glucose transporter GLUT3 (see Figure 3 of Vaeth & Feske). Klein-Hessling et al., *supra*, also showed that NFAT2 controls metabolic gene expression, the glycolytic switch, and thus the function of cytotoxic CD8[+] T cells.

NFAT is also critical for the function and differentiation of T helper (Th) cells, including Th1, Th2, and Th17 cells. NFAT proteins differentially affect Th cell differentiation (reviewed in Lee et al., Frontiers in Immunology, 2018, Vol. 9, Article 2747 - see in particular Figure 2 of this document). Each differentiated T subset is characterized by the expression of their specific master regulator transcription factors and signature cytokines. Differentiation of each Th cell is initiated by activation of T cell receptor (TCR), costimulatory receptor signals (e.g., CD28 or ICOS), and specific lineage-determining cytokine signals. These signals induce the NFAT/AP-1 complex to express lineage-related transcription factors such as T-bet for Th1, GATA3 for Th2, RORγt for Th17, and BATF for follicular helper T (Tfh) cells. In combination with these transcription factors, NFAT/partner protein complexes determine their lineage differentiation and functional characteristics (surface receptors and cytokine production).

Optionally the one or more NFAT-regulated genes include genes encoding cytokines, receptors, or nuclear factors, such as genes encoding IL-2, IFN-y, IL-4, IL-6, IL-17, IL-21, IL-22, IL-23, CD25, PD-1, TIM-3.

In particular, the one or more NFAT-regulated genes may include a gene encoding IL-2.

IL-2 influences effector T cell differentiation and is a critical determinant of the fate decisions of antigen receptor-activated T cells (reviewed in Ross & Cantrell, Annual Review of Immunology, 2018, 36:411-33). IL-2 can promote Th1 and Th2 fate decisions in antigen receptor-activated CD4$^+$ T cells but suppress the differentiation of Th17 cells and Tfh cells. IL-2 controls CD4$^+$ T cell fate by controlling the expression of the key cytokine receptors, transcription factors, chromatin regulators, and effector cytokines. For example, IL-2 can induce the expression of Eomesodermin, T-bet (Tbx21), and Blimp-1 while suppressing BCL6. Additionally, IL-2 can regulate how cells may respond to other proinflammatory cytokines by stimulating the expression of the IL-12R, IL-12Rβ1, and IL-12Rβ2 chains and the IL-4Rα chain, and suppressing the expression of IL-6Rα and gp130 and IL-7R.

IL-2 is also important for CD8$^+$ effector cells. In response to infection, naïve antigen-specific CD8+ T cells proliferate and differentiate into effector cytotoxic T lymphocytes (CTLs) that make proinflammatory cytokines such as IFN-γ and acquire the ability to kill infected cells. IL-2 can influence these effector activities in CD8$^+$ T cells by inducing the expression of IFN-γ, TNF-α, and lymphotoxin α, by stimulating expression of the cytolytic effector molecules granzyme B and perforin, and by promoting effective target cell killing. IL-2 also acts to control the size of CTLs by regulating amino acid uptake and protein synthesis.

The term "LAG-3 positive T cells" is used herein to refer to LAG-3 activated CD4$^+$ and CD8$^+$ T lymphocytes on which LAG-3 is expressed. Expression of LAG-3 may be determined, for example, by use of a labelled anti-LAG-3 antibody.

Inhibition of NFAT-regulated gene expression in LAG-3 positive T cells using a binding molecule of the invention is highly advantageous because such binding molecules inhibit NFAT-regulated gene expression without directly inhibiting calcineurin, thereby avoiding the serious side effects of calcineurin inhibitors. Furthermore, since the binding molecule is an agonist of LAG-3, inhibition of NFAT-regulated gene expression is limited to LAG-3 positive cells. Since LAG-3 is expressed primarily on activated CD4$^+$ and CD8$^+$ T cells, and NK cells, the effects of the binding molecule are largely targeted to activated T cells, thereby minimising any non-T cell-mediated side effects.

Optionally a binding molecule of the invention inhibits antigen-induced CD4$^+$ and/or CD8$^+$ T-cell proliferation, in particular by inhibition of expression of one or more NFAT-regulated genes, for example a gene encoding IL-2, in LAG-3 positive T-cells.

Optionally a binding molecule of the invention inhibits antigen-induced CD4$^+$ and/or CD8$^+$ T-cell activation, in particular by inhibition of expression of one or more NFAT-regulated genes, for example a gene encoding IL-2, in LAG-3 positive T-cells.

A binding molecule of the invention may be used for the treatment of a T cell-mediated immune disorder, in particular by inhibition of expression of one or more NFAT-regulated genes, for example a gene encoding IL-2, in LAG-3 positive T-cells.

The term "LAG-3" used herein refers to Lymphocyte Activation Gene-3. The term "LAG-3" includes variants, isoforms, homologs, orthologs and paralogs. For example, binding molecules specific for a human LAG-3 protein may, in certain cases, cross-react with a LAG-3 protein from a species other than human. In other embodiments, the binding molecules specific for a human LAG-3 protein may be completely specific for the human LAG-3 protein and may not exhibit species or other types of cross-reactivity, or may cross-react with LAG-3 from certain other species but not all other species (e.g., cross-react with monkey LAG-3 but not mouse LAG-3).

The amino acid sequence of human and murine LAG-3 protein is provided in Figure 1 of Huard et al (Proc. Natl. Acad. Sci. USA, 11: 5744-5749, 1997). The sequence of human LAG-3 protein is repeated in Figure 3 below (SEQ ID NO:13). The amino acid sequences of the four extracellular Ig superfamily domains (D1, D2, D3, and D4) of human LAG-3 are at amino acid residues: 1-149 (D1) (SEQ ID NO:14); 150-239 (D2) (SEQ ID NO:15); 240-330 (D3) (SEQ ID NO:16); and 331-412 (D4) (SEQ ID NO:17). Huard *et al* (*Proc. Natl. Acad. Sci.* USA, **11**: 5744-5749, 1997) describes characterization of the MHC class II binding site on LAG-3 protein. Many of the residues essential for binding MHC class II proteins are clustered at the base of a large 30 amino acid extra-loop structure in the LAG-3 D1 domain. The amino acid sequence of the extra-loop structure of the D1 domain of human LAG-3 protein is GPPAAAPGHPLAPGPHPAAPSSWGPRPRRY (SEQ ID NO:18), the underlined sequence in Figure 3.

The term "human LAG-3" refers to human sequence LAG-3, such as the complete amino acid sequence of human LAG-3 having Genbank Accession No. NP 002277 (SEQ ID NO: 22), or the amino acid sequence of human LAG-3 protein given in Figure 3 (SEQ ID NO: 13). The term "mouse LAG-3" refers to mouse sequence LAG-3, such as the complete amino acid sequence of mouse LAG-3 having Genbank Accession No. NP_032505. LAG-3 is also known in the art as, for example, CD223. The human LAG-3 sequence may differ from human LAG-3 of Genbank Accession No. NP_002277 by having, e.g., conserved mutations or mutations in non-conserved regions and the LAG-3 has substantially the same biological function as the human LAG-3 of Genbank Accession No. NP_002277. For example, a biological function of human LAG-3 is having a discontinuous epitope in the extracellular D1 domain of LAG-3 that is specifically bound by a binding molecule of the instant disclosure or a biological function of human LAG-3 is binding to MHC Class II molecules. The term "monkey LAG-3" is intended to encompass LAG-3 proteins expressed by Old World and New World monkeys, including but not limited to cynomolgus monkey LAG-3 and rhesus monkey LAG-3. A representative amino acid sequence for monkey LAG-3 is the rhesus monkey LAG-3 amino acid sequence which is also deposited as Genbank Accession No. XM_001108923. Another representative amino acid sequence for monkey LAG-3 is the alternative rhesus monkey sequence of clone pa23-5 as described in US 2011/0150892 A1. This alternative rhesus sequence exhibits a single amino acid difference, at position 419, as compared to the Genbank-deposited sequence.

A particular human LAG-3 sequence will generally be at least 90% identical in amino acid sequence to human LAG-3 of Genbank Accession No. NP_002277 and contains amino acid residues that identify the amino acid sequence as being human when compared to LAG-3 amino acid sequences of other species (e.g., murine). In certain cases, a human LAG-3 can be at least 95%, or even at least 96%, 97%, 98%, or 99% identical in amino acid sequence to LAG-3 of Genbank Accession No. NP_002277. In certain embodiments, a human LAG-3 sequence will display no more than 10 amino acid differences from the LAG-3 sequence of Genbank Accession No. NP_002277. In certain embodiments, the human LAG-3 can display no more than 5, or even no more than 4, 3, 2, or 1 amino acid difference from the LAG-3 sequence of Genbank Accession No. NP_002277.

Optionally a binding molecule of the invention binds specifically to a discontinuous epitope within the extracellular Ig superfamily domain D1 (SEQ ID NO:14) of a LAG-3 protein, wherein amino acid residues of the discontinuous epitope lie outside a 30 amino acid extra-loop sequence (SEQ ID NO:18) of the domain D1 of the LAG-3 protein.

According to the invention, there is also provided an isolated binding molecule, which binds specifically to a discontinuous epitope within the extracellular Ig superfamily domain D1 (SEQ ID NO:14) of a LAG-3 protein, wherein amino acid residues of the discontinuous epitope lie outside a 30 amino acid extra-loop sequence (SEQ ID NO:18) of the domain D1 of the LAG-3 protein.

Optionally the binding molecule is an agonist of LAG-3.

The term "discontinuous epitope" is used herein to refer to an epitope comprising sequences of amino acid residues that are distantly placed in the LAG-3 amino acid sequence, but are juxtaposed in the three-dimensional structure of the LAG-3 protein, enabling the binding molecule to recognize the protein in its native conformation. A "discontinuous epitope" is also referred to as a "conformational epitope".

Optionally the discontinuous epitope comprises any of the following amino acid sequences:

|  |  |
|---|---|
| TIPLQDLSLLRR | (SEQ ID NO:19); |
| GGLRSGRLPLQ | (SEQ ID NO:20); |
| QRGDFSLWLRPAR | (SEQ ID NO:21); |
| LRDRALSCRL | (SEQ ID NO:22). |

Optionally the discontinuous epitope comprises two or more of the following amino acid sequences:

|  |  |
|---|---|
| TIPLQDLSLLRR | (SEQ ID NO:19); |
| GGLRSGRLPLQ | (SEQ ID NO:20); |
| QRGDFSLWLRPAR | (SEQ ID NO:21); |
| LRDRALSCRL | (SEQ ID NO:22). |

Optionally the discontinuous epitope comprises three or more of the following amino acid sequences:

|  |  |
|---|---|
| TIPLQDLSLLRR | (SEQ ID NO:19); |
| GGLRSGRLPLQ | (SEQ ID NO:20); |
| QRGDFSLWLRPAR | (SEQ ID NO:21); |
| LRDRALSCRL | (SEQ ID NO:22). |

Optionally the discontinuous epitope comprises the following amino acid sequences:

|  |  |
|---|---|
| TIPLQDLSLLRR | (SEQ ID NO:19); |
| GGLRSGRLPLQ | (SEQ ID NO:20); |

(continued)

QRGDFSLWLRPAR     (SEQ ID NO:21);
LRDRALSCRL     (SEQ ID NO:22).

and

A binding molecule of the invention may be any type of molecule, including a biological molecule, such as a protein, or a chemical molecule, such as a synthetic small molecule. Optionally a binding molecule of the invention comprises an antibody, or an antigen-binding fragment thereof.

Optionally a binding molecule of the invention binds specifically to the discontinuous epitope within a LAG-3 protein, particularly a human LAG-3 protein. "Specific binding" refers to binding with an affinity of at least about $5x10^{-7}$ M or greater, e.g., $10^{-7}$ M, $5 \times 10^{-8}$ M, $10^{-8}$ M, or greater. "Non-specific binding" refers to binding with an affinity of less than about $10^{-7}$ M, e.g., binding with an affinity of $10^{-6}$ M, $10^{-5}$ M, or $10^{-4}$ M. As used herein, a binding molecule that "specifically binds human LAG-3" is intended to refer to a binding molecule that binds to human LAG-3 protein (and possibly a LAG-3 protein from one or more non-human species) but does not substantially bind to non-LAG-3 proteins. Preferably, the binding molecule binds to a human LAG-3 protein with "high affinity", namely with a $K_D$ of $1x10^{-7}$ M or less, more preferably $1x10^{-8}$ M or less, more preferably $5x10^{-9}$ M or less, more preferably $1x10^{-9}$ M or less.

As used herein, the term "affinity" refers to the equilibrium constant for the reversible binding of two agents (e.g., a binding molecule of the invention for LAG-3 protein) and is expressed as a dissociation constant ($K_D$). Affinity can be at least 1-fold greater, at least 2-fold greater, at least 3-fold greater, at least 4-fold greater, at least 5-fold greater, at least 6-fold greater, at least 7-fold greater, at least 8-fold greater, at least 9-fold greater, at least 10-fold greater, at least 20-fold greater, at least 30-fold greater, at least 40-fold greater, at least 50-fold greater, at least 60-fold greater, at least 70-fold greater, at least 80-fold greater, at least 90-fold greater, at least 100-fold greater, or at least 1,000-fold greater, or more, than the affinity of the binding molecule for unrelated amino acid sequences. Affinity of a binding molecule of the invention for LAG-3 protein can be, for example, from about 100 nanomolar (nM) to about 0.1 nM, from about 100 nM to about 1 picomolar (pM), or from about 100 nM to about 1 femtomolar (fM) or more.

The term "does not substantially bind" means does not bind or does not bind with a high affinity to non-LAG-3 proteins, i.e. binds to such proteins with a $K_D$ of $1x10^{-6}$ M or more, more preferably $1 \times 10^{-5}$ M or more, more preferably $1x10^{-4}$ M or more, more preferably $1x10^{-3}$ M or more, even more preferably $1x10^{-2}$ M or more.

The term "$K_{assoc}$" or "$K_a$", as used herein, is intended to refer to the association rate of a particular binding molecule-binding partner, whereas the term "$K_{dis}$" or "$K_d$," as used herein, is intended to refer to the dissociation rate of a particular binding molecule-binding partner interaction. The term "$K_D$" as used herein, is intended to refer to the dissociation constant, which is obtained from the ratio of $K_d$ to $K_a$. (i.e., $K_d/K_a$) and is expressed as a molar concentration (M).

The term "high affinity" for an IgG antibody refers to an antibody having a $K_D$ of $1x10^{-7}$ M or less, more preferably $5x10^{-8}$ M or less, even more preferably $1x10^{-8}$ M or less, even more preferably $5x10^{-9}$ M or less and even more preferably $1x10^{-9}$ M or less for a target antigen. However, "high affinity" binding can vary for other antibody isotypes. For example, "high affinity" binding for an IgM isotype refers to an antibody having a $K_D$ of $10^{-6}$ M or less, more preferably $10^{-7}$ M or less, even more preferably $10^{-8}$ M or less.

In some embodiments, a binding molecule of the present invention binds a human LAG-3 protein (or a derivative thereof, such as a human LAG-3Ig protein) with a dissociation constant ($K_D$) of no more than 3.5 nM, no more than 2.5 nM, no more than 2 nM, no more than 1 nM, no more than 0.9 nM, no more than 0.8 nM, no more than 0.7 nM, no more than 0.6 nM, no more than 0.5 nM, no more than 0.4 nM, no more than 0.3 nM, no more than 0.2 nM, no more than 0.1 nM. In some embodiments, a binding molecule of the present disclosure binds a human LAG-3 protein with a $K_D$ of no more than 90 pM, no more than 80 pM, no more than 70 pM, no more than 60 pM, no more than 50 pM, no more than 40 pM, no more than 30 pM, no more than 20 pM, no more than 10 pM, no more than 9 pM, no more than 8 pM, no more than 7 pM, no more than 6 pM, no more than 5 pM, no more than 4 pM, no more than 3 pM, no more than 2 pM, or no more than 1 pM.

The affinity of a binding molecule to a human LAG-3 protein can be determined by one skilled in the art, suitably by surface plasmon resonance, for example using a biosensor system, such as a Biacore system (Murphy et al, Using Biacore to measure the binding kinetics of an antibody-antigen interaction; Curr Protoc Protein Sci. 2006 Sep;Chapter 19:Unit 19.14). For example, Biacore analysis can be used to determine the dissociation constant between a binding molecule of the invention and a human LAG-3 protein.

Example 20 of WO 2017/037203 describes the results of Biacore analysis of binding of chimeric 13E2 IgG4 antibody (Chim13E2IgG4), and of humanized 13E2 IgG4 (IMP761), to human LAG-3Ig protein. The results show that the dissociation constant of chimeric 13E2 IgG4 antibody for human LAG-3Ig was 21.9 pM, and of humanized 13E2 IgG4 for human LAG-3Ig was 22.8 pM.

In particular embodiments, a binding molecule of the present invention binds a human LAG-3 protein (or a human LAG-3Ig protein) with a dissociation constant ($K_D$) of no more than 100pM, no more than 90 pM, no more than 80 pM, no more than 70 pM, no more than 60 pM, no more than 50 pM, no more than 40 pM, no more than 30 pM, or no more than 25 pM, for example as determined by Biacore analysis.

Binding to human LAG-3 can be assessed using one or more other techniques also well established in the art. For example, a binding molecule can be tested by a flow cytometry assay in which the binding molecule is reacted with a cell line that expresses human LAG-3, such as CHO cells or Jurkat cells that have been transfected to express human LAG-3 on their cell surface. Other suitable cells for use in flow cytometry assays include SEB-stimulated PBMCs, or anti-CD3-stimulated CD4$^+$ activated T cells, which express native LAG-3. Still other suitable binding assays include ELISA assays, for example, using a recombinant LAG-3 protein.

A binding molecule of the invention is an isolated binding molecule. An "isolated" binding molecule is a binding molecule that has been identified and separated and/or recovered from a component of its natural environment. Contaminant components of its natural environment are materials that would interfere with diagnostic or therapeutic uses for the binding molecule, and can include enzymes, hormones, and other proteinaceous or non-proteinaceous solutes. In some embodiments, where the binding molecule is a protein, it will be purified (1) to greater than 90%, greater than 95%, or greater than 98%, by weight of the molecule, for example as determined by the Lowry method (Lowry, et al (1951). "Protein measurement with the Folin phenol reagent". Journal of Biological Chemistry. 193 (1): 265-75), for example, more than 99% by weight, (2) to a degree sufficient to obtain at least 15 residues of N-terminal or internal amino acid sequence by use of a spinning cup sequenator, or (3) to homogeneity by sodium dodecyl sulfate-polyacrylamide gel electrophoresis (SDS-PAGE) under reducing or nonreducing conditions using Coomassie blue or silver stain. Where the isolated binding molecule is a protein (such as an antibody or fragment thereof), this includes the protein *in situ* within recombinant cells since at least one component of the protein's natural environment will not be present. In some instances, isolated binding molecule will be prepared by at least one purification step.

Optionally a binding molecule of the invention comprises one, two, or three complementarity determining regions (CDRs) of an antibody heavy chain variable (VH) region of IMP761 (see Table 1: VH CDRs1-3; SEQ ID NOs:1-3, or 7-9), and/or one, two, or three CDRs of an antibody light chain variable (VL) region of IMP761 (see Table 1: VL CDRs1-3; SEQ ID NOs:4-6, or 10-12).

Antibodies 13E2 and IMP761 comprise VH CDR1-3 and VL CDR1-3 sequences as set out in Table 1 below:

Table 1. Anti-LAG-3 Agonist Antibody VH and VL CDR Sequences

| Antibody | CDR-1 | CDR-2 | CDR-3 |
|---|---|---|---|
| **13E2 VH** (IMGT numbering) | GFSLSTSGMG (SEQ ID NO:1) | IWWDDIK (SEQ ID NO:2) | ARIVEGSYSSSYFDV (SEQ ID NO:3) |
| **13E2 VL** (IMGT numbering) | QDVIFD (SEQ ID NO:4) | SAS (SEQ ID NO:5) | QQHYSTPYT (SEQ ID NO:6) |
| **13E2 VH** (Kabat numbering) | TSGMGLG (SEQ ID NO:7) | HIWWDDIKRYNPDLRS (SEQ ID NO:8) | IVEGSYSSSYFDV (SEQ ID NO:9) |
| **13E2 VL** (Kabat numbering) | KASQDVIFDVA (SEQ ID NO:10) | SASSRVS (SEQ ID NO:11) | QQHYSTPYT (SEQ ID NO:12) |

Optionally a binding molecule of the invention is a variant of antibody 13E2 or IMP761. For example, amino acid residues within the VH and/or VL CDR1, CDR2 and/or CDR3 regions may be mutated relative to the corresponding region of the 13E2 or IMP761 antibodies. Site-directed mutagenesis or PCR-mediated mutagenesis can be performed to introduce the mutation(s) and the effect on antibody binding, or other functional property of interest, can be evaluated in *in vitro* or *in vivo* assays as described herein and provided in Example 1 below, or in the Examples in WO 2017/037203. Preferably, conservative modifications are introduced. The mutations can be amino acid substitutions, additions or deletions, but are preferably substitutions. Moreover, typically no more than one, two, three, four or five residues within a CDR region are altered. In some embodiments, no more than one, two, three, four or five residues are altered in total for all six CDR regions.

In certain embodiments, a binding molecule of the invention comprises an antibody (or antigen-binding fragment thereof) comprising a heavy chain variable region comprising CDR1, CDR2 and CDR3 sequences, and a light chain variable region comprising CDR1, CDR2, and CDR3 sequences, which differ from those of antibody 13E2 or IMP761 by one or more conservative modifications, for example up to five conservative modifications.

It is understood in the art that certain conservative sequence modifications can be made which do not remove antigen binding. See, e.g., Brummell et al. (1993) Biochem 32:1180-8; de Wildt et al. (1997) Prot. Eng. 10:835-41; Komissarov et al. (1997) J. Biol. Chem. 272:26864-26870; Hall et al. (1992) J. Immunol. 149:1605-12; Kelley and O'Connell (1993) Biochem. 32:6862-35; Adib-Conquy et al. (1998) Int. Immunol. 10:341-6 and Beers et al. (2000) Clin.

Can. Res. 6:2835-43.

As used herein, the term "conservative sequence modifications" refers to amino acid modifications that do not significantly affect or alter the binding characteristics of the binding molecule containing the amino acid sequence. Such conservative modifications include amino acid substitutions, additions and deletions. Modifications can be introduced by standard techniques known in the art, such as site-directed mutagenesis and PCR-mediated mutagenesis. Conservative amino acid substitutions are ones in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine, tryptophan), nonpolar side chains (e.g., alanine, valine, leucine, isoleucine, proline, phenylalanine, methionine), beta-branched side chains (e.g., threonine, valine, isoleucine) and aromatic side chains (e.g., tyrosine, phenylalanine, tryptophan, histidine). Thus, one or more amino acid residues (for example no more than two, three, four, or five amino acid residues) within the CDR regions can be replaced with other amino acid residues from the same side chain family and the altered antibody can be tested for retained function (i.e., the functions set forth above) using the functional assays described herein (or in WO 2017/037203).

Optionally a binding molecule of the invention comprises an antibody, or an antigen-binding fragment thereof, comprising: an antibody VH region comprising: a VH CDR1 with an amino acid sequence selected from SEQ ID NO:1 and 7; a VH CDR2 with an amino acid sequence selected from SEQ ID NO:2 and 8; and a VH CDR3 with an amino acid sequence selected from SEQ ID NO:3 and 9; and an antibody VL region comprising: a VL CDR1 with an amino acid sequence selected from SEQ ID NO:4 and 10; a VL CDR2 with an amino acid sequence selected from SEQ ID NO:5 and 11; and a VL CDR3 with an amino acid sequence selected from SEQ ID NO:6 and 12; or a variant thereof in which no more than one, two, three, four or five amino acid residues are altered by amino acid substitution, addition, or deletion, within the CDR sequences.

Optionally a binding molecule of the invention comprises an antibody, or an antigen-binding fragment thereof, comprising a heavy chain variable region comprising: (a) a VH CDR1 region comprising SEQ ID NO:1, or an amino acid sequence having one, two, three, four or five amino acid substitutions, deletions or additions as compared to SEQ ID NO: 1; (b) a VH CDR2 region comprising SEQ ID NO:2, or an amino acid sequence having one, two, three, four or five amino acid substitutions, deletions or additions as compared to SEQ ID NO:2; and (c) a VH CDR3 region comprising SEQ ID NO:3, or an amino acid sequence having one, two, three, four or five amino acid substitutions, deletions or additions as compared to SEQ ID NO:3; and/or a light chain variable region comprising: (a) a VL CDR1 region comprising SEQ ID NO:4, or an amino acid sequence having one, two, three, four or five amino acid substitutions, deletions or additions as compared to SEQ ID NO:4; (b) a VL CDR2 region comprising SEQ ID NO:5, or an amino acid sequence having one, two, three, four or five amino acid substitutions, deletions or additions as compared to SEQ ID NO:5; and (c) a VL CDR3 region comprising SEQ ID NO:6, or an amino acid sequence having one, two, three, four or five amino acid substitutions, deletions or additions as compared to SEQ ID NO:6.

Optionally a binding molecule of the invention comprises an antibody, or an antigen-binding fragment thereof, comprising a heavy chain variable region comprising: (a) a VH CDR1 region comprising SEQ ID NO:7, or an amino acid sequence having one, two, three, four or five amino acid substitutions, deletions or additions as compared to SEQ ID NO:7; (b) a VH CDR2 region comprising SEQ ID NO:8, or an amino acid sequence having one, two, three, four or five amino acid substitutions, deletions or additions as compared to SEQ ID NO:8; and (c) a VH CDR3 region comprising SEQ ID NO:9, or an amino acid sequence having one, two, three, four or five amino acid substitutions, deletions or additions as compared to SEQ ID NO:9; and/or a light chain variable region comprising: (a) a VL CDR1 region comprising SEQ ID NO:10, or an amino acid sequence having one, two, three, four or five amino acid substitutions, deletions or additions as compared to SEQ ID NO:10; (b) a VL CDR2 region comprising SEQ ID NO:11, or an amino acid sequence having one, two, three, four or five amino acid substitutions, deletions or additions as compared to SEQ ID NO:11; and (c) a VL CDR3 region comprising SEQ ID NO:12, or an amino acid sequence having one, two, three, four or five amino acid substitutions, deletions or additions as compared to SEQ ID NO:12.

Optionally a binding molecule of the invention excludes an antibody, or antigen-binding fragment thereof, that comprises VH CDRs1-3 and VL CDRs1-3 of antibody IMP761 (see Table 1: VH CDRs1-3; SEQ ID NOs:1-3, or 7-9; VL CDRs1-3; SEQ ID NOs:4-6, or 10-12).

Optionally a binding molecule of the invention excludes IMP761, or an antigen-binding fragment thereof that is an agonist of LAG-3.

Optionally a binding molecule of the invention excludes 13E2, or an antigen-binding fragment thereof that is an agonist of LAG-3.

Optionally a binding molecule of the invention excludes an antibody, or antigen-binding fragment thereof, that comprises any of VH CDRs1-3 and VL CDRs1-3 of antibody IMP761.

Optionally a binding molecule of the invention excludes an antibody, or antigen-binding fragment thereof, that comprises any of VH CDRs1-3 (SEQ ID NOs:1-3 or 7-9) and VL CDRs1-3 (SEQ ID NOs:4-6 or 10-12) of antibody IMP761.

Optionally a binding molecule of the invention excludes an antibody, or antigen-binding fragment thereof, that comprises one, two, or three complementarity determining regions (CDRs) of an antibody heavy chain variable (VH) region of IMP761 (VH CDRs1-3: SEQ ID NOs:1-3 or 7-9), and/or one, two, or three CDRs of an antibody light chain variable (VL) region of IMP761 (VL CDRs1-3: SEQ ID NOs:4-6 or 10-12).

Optionally a binding molecule of the invention excludes an antibody, or antigen-binding fragment thereof, that comprises: an antibody VH region comprising: a VH CDR1 with an amino acid sequence selected from SEQ ID NO:1 and 7; a VH CDR2 with an amino acid sequence selected from SEQ ID NO:2 and 8; and a VH CDR3 with an amino acid sequence selected from SEQ ID NO:3 and 9; and an antibody VL region comprising: a VL CDR1 with an amino acid sequence selected from SEQ ID NO:4 and 10; a VL CDR2 with an amino acid sequence selected from SEQ ID NO:5 and 11; and a VL CDR3 with an amino acid sequence selected from SEQ ID NO:6 and 12; or a variant thereof in which no more than one, two, three, four or five amino acid residues are altered by amino acid substitution, addition, or deletion, within the CDR sequences.

Optionally a binding molecule of the invention excludes an antibody, or antigen-binding fragment thereof, that comprises: a heavy chain variable region comprising: (a) a VH CDR1 region comprising SEQ ID NO: 1, or an amino acid sequence having one, two, three, four or five amino acid substitutions, deletions or additions as compared to SEQ ID NO: 1 ; (b) a VH CDR2 region comprising SEQ ID NO: 2, or an amino acid sequence having one, two, three, four or five amino acid substitutions, deletions or additions as compared to SEQ ID NO: 2; and (c) a VH CDR3 region comprising SEQ ID NO: 3, or an amino acid sequence having one, two, three, four or five amino acid substitutions, deletions or additions as compared to SEQ ID NO: 3; and/or a light chain variable region comprising: (a) a VL CDR1 region comprising SEQ ID NO: 4, or an amino acid sequence having one, two, three, four or five amino acid substitutions, deletions or additions as compared to SEQ ID NO: 4; (b) a VL CDR2 region comprising SEQ ID NO: 5, or an amino acid sequence having one, two, three, four or five amino acid substitutions, deletions or additions as compared to SEQ ID NO: 5; and (c) a VL CDR3 region comprising SEQ ID NO: 6, or an amino acid sequence having one, two, three, four or five amino acid substitutions, deletions or additions as compared to SEQ ID NO: 6.

Optionally a binding molecule of the invention excludes an antibody, or antigen-binding fragment thereof, that comprises: a heavy chain variable region comprising: (a) a VH CDR1 region comprising SEQ ID NO: 7, or an amino acid sequence having one, two, three, four or five amino acid substitutions, deletions or additions as compared to SEQ ID NO: 7; (b) a VH CDR2 region comprising SEQ ID NO: 8, or an amino acid sequence having one, two, three, four or five amino acid substitutions, deletions or additions as compared to SEQ ID NO: 8; and (c) a VH CDR3 region comprising SEQ ID NO: 9, or an amino acid sequence having one, two, three, four or five amino acid substitutions, deletions or additions as compared to SEQ ID NO: 9; and/or a light chain variable region comprising: (a) a VL CDR1 region comprising SEQ ID NO: 10, or an amino acid sequence having one, two, three, four or five amino acid substitutions, deletions or additions as compared to SEQ ID NO: 10; (b) a VL CDR2 region comprising SEQ ID NO: 11, or an amino acid sequence having one, two, three, four or five amino acid substitutions, deletions or additions as compared to SEQ ID NO: 11; and (c) a VL CDR3 region comprising SEQ ID NO: 12, or an amino acid sequence having one, two, three, four or five amino acid substitutions, deletions or additions as compared to SEQ ID NO: 12.

WO 2017/037203 also describes mouse monoclonal anti-LAG-3 antibody 34F4. Antibody 34F4 comprises VH CDR1-3 and VL CDR1-3 sequences as set out in Table 2 below:

Table 2. 34F4 VH and VL CDR Sequences

| Antibody | CDR-1 | CDR-2 | CDR-3 |
|---|---|---|---|
| **34F4 VH** (IMGT numbering) | GFSLNTSGMG (SEQ ID NO:86) | IWWDDVK (SEQ ID NO:87) | ARIEGDTYYDYYFDY (SEQ ID NO:88) |
| **34F4 VL** (IMGT numbering) | QDVSIA (SEQ ID NO:89) | SAS (SEQ ID NO:90) | QQHYSIPWT (SEQ ID NO:91) |
| **34F4 VH** (Kabat numbering) | TSGMGVG (SEQ ID NO:92) | HIWWDDVKRYNPALKS (SEQ ID NO:93) | IEGDTYYDYYFDY (SEQ ID NO:94) |
| **34F4 VL** (Kabat numbering) | KASQDVSIAVV (SEQ ID NO:95) | SASFRYT (SEQ ID NO:96) | QQHYSIPWT (SEQ ID NO:97) |

Optionally a binding molecule of the invention excludes an antibody, or antigen-binding fragment thereof, that comprises VH CDRs1-3 and VL CDRs1-3 of antibody 34F4 (see Table 2: VH CDRs1-3; SEQ ID NOs:86-88, or 92-94; VL CDRs1-3; SEQ ID NOs:89-91, or 95-97).

Optionally a binding molecule of the invention excludes antibody 34F4, or an antigen-binding fragment thereof that is an agonist of LAG-3.

**EP 4 609 916 A2**

Optionally a binding molecule of the invention excludes an antibody, or antigen-binding fragment thereof, that comprises any of VH CDRs1-3 (SEQ ID NOs:86-88 or 92-94) and VL CDRs1-3 (SEQ ID NOs:89-91 or 95-97) of antibody 34F4.

Optionally a binding molecule of the invention excludes an antibody, or antigen-binding fragment thereof, that comprises one, two, or three complementarity determining regions (CDRs) of an antibody heavy chain variable (VH) region of IMP761 (VH CDRs1-3: SEQ ID NOs:86-88 or 92-94), and/or one, two, or three CDRs of an antibody light chain variable (VL) region of IMP761 (VL CDRs1-3: SEQ ID NOs: 89-91 or 95-97).

Optionally a binding molecule of the invention excludes an antibody, or antigen-binding fragment thereof, that comprises: an antibody VH region comprising: a VH CDR1 with an amino acid sequence selected from SEQ ID NO:86 and 92; a VH CDR2 with an amino acid sequence selected from SEQ ID NO:87 and 93; and a VH CDR3 with an amino acid sequence selected from SEQ ID NO:88 and 94; and an antibody VL region comprising: a VL CDR1 with an amino acid sequence selected from SEQ ID NO:89 and 95; a VL CDR2 with an amino acid sequence selected from SEQ ID NO:90 and 96; and a VL CDR3 with an amino acid sequence selected from SEQ ID NO:91 and 97; or a variant thereof in which no more than one, two, three, four or five amino acid residues are altered by amino acid substitution, addition, or deletion, within the CDR sequences.

Optionally a binding molecule of the invention excludes an antibody, or antigen-binding fragment thereof, that comprises: a heavy chain variable region comprising: (a) a VH CDR1 region comprising SEQ ID NO:86, or an amino acid sequence having one, two, three, four or five amino acid substitutions, deletions or additions as compared to SEQ ID NO:86; (b) a VH CDR2 region comprising SEQ ID NO:87, or an amino acid sequence having one, two, three, four or five amino acid substitutions, deletions or additions as compared to SEQ ID NO:87; and (c) a VH CDR3 region comprising SEQ ID NO:88, or an amino acid sequence having one, two, three, four or five amino acid substitutions, deletions or additions as compared to SEQ ID NO:88; and/or a light chain variable region comprising: (a) a VL CDR1 region comprising SEQ ID NO:89, or an amino acid sequence having one, two, three, four or five amino acid substitutions, deletions or additions as compared to SEQ ID NO:89; (b) a VL CDR2 region comprising SEQ ID NO:90, or an amino acid sequence having one, two, three, four or five amino acid substitutions, deletions or additions as compared to SEQ ID NO:90; and (c) a VL CDR3 region comprising SEQ ID NO:91, or an amino acid sequence having one, two, three, four or five amino acid substitutions, deletions or additions as compared to SEQ ID NO:91.

Optionally a binding molecule of the invention excludes an antibody, or antigen-binding fragment thereof, that comprises: a heavy chain variable region comprising: (a) a VH CDR1 region comprising SEQ ID NO:92, or an amino acid sequence having one, two, three, four or five amino acid substitutions, deletions or additions as compared to SEQ ID NO:92; (b) a VH CDR2 region comprising SEQ ID NO:93, or an amino acid sequence having one, two, three, four or five amino acid substitutions, deletions or additions as compared to SEQ ID NO:93; and (c) a VH CDR3 region comprising SEQ ID NO:94, or an amino acid sequence having one, two, three, four or five amino acid substitutions, deletions or additions as compared to SEQ ID NO:94; and/or a light chain variable region comprising: (a) a VL CDR1 region comprising SEQ ID NO:95, or an amino acid sequence having one, two, three, four or five amino acid substitutions, deletions or additions as compared to SEQ ID NO: 95; (b) a VL CDR2 region comprising SEQ ID NO:96, or an amino acid sequence having one, two, three, four or five amino acid substitutions, deletions or additions as compared to SEQ ID NO:96; and (c) a VL CDR3 region comprising SEQ ID NO:97, or an amino acid sequence having one, two, three, four or five amino acid substitutions, deletions or additions as compared to SEQ ID NO:97.

Optionally a binding molecule of the invention is an agonist of human LAG-3.

Agonist activity of a binding molecule of the invention may be determined by any suitable method. An example of a suitable *in vitro* method is described in Example 1 below, using IMP761 as agonist. According to this method, a binding molecule of the invention is considered to have LAG-3 agonist activity if the $IC_{50}$ of the binding molecule for LAG-3 is 1 mM or less, 1 $\mu$M or less, or 1 nM or less, for example $10^{-9}$ to $10^{-10}$ M (~50 ng/ml).

Differences between depleting anti-LAG-3 antibodies, antagonist anti-LAG-3 antibodies, and agonistic anti-LAG-3 antibodies are described in WO 2017/037203. A depleting anti-LAG-3 antibody causes depletion of activated T cells by binding to LAG-3 expressed on the surface of the cells. Depletion can occur by antibody-dependent cell-mediated cytotoxicity (ADCC), or by complement-dependent cytotoxicity (CDC). In ADCC, the Fc region of the depleting antibody binds to Fc receptors (FcyRs) on the surface of immune effector cells, such as natural killers and macrophages, leading to lysis of the targeted cells. In CDC, the Fc region of the depleting antibody binds to the C1q component of complement, and the targeted cell is killed by triggering the complement cascade at the cell surface. Thus, depleting anti-LAG-3 antibodies inhibit T cell-mediated immune responses. It will be appreciated that the effects of depleting anti-LAG-3 antibodies are long-lasting, and irreversible because they cause the destruction of activated T cells. Such antibodies are useful, for example, for the treatment of inflammatory and autoimmune disorders, and for the prevention of transplant rejection

An antagonist anti-LAG-3 antibody binds to LAG-3 on the surface of activated T cells, and prevents interaction of LAG-3 with MHC class II molecules on the surface of antigen-presenting cells (APCs). This blocks the negative regulation of signal transduction that occurs when APCs bind to LAG-3 on activated T cells. Consequently, antagonist anti-LAG-3 antibodies prevent the negative regulation of T cell proliferation, function and homeostasis normally mediated by LAG-3.

Such antibodies are useful, for example, for the treatment of cancer and infectious disease.

Agonistic anti-LAG-3 antibodies bind to LAG-3 on the surface of activated T cells, and negatively regulate signal transduction through agonism of LAG-3 (in particular, by inhibiting expression of NFAT-regulated genes, such as a gene encoding IL-2), causing negative regulation of T cell proliferation and/or activation. Thus, agonistic anti-LAG-3 antibodies inhibit T cell-mediated immune responses, in particular by inhibiting antigen-induced CD4$^+$ and/or CD8$^+$ T cell proliferation, and/or antigen-induced CD4$^+$ and/or CD8$^+$ T cell activation. The effects of such antibodies are reversible, and may be less long-lasting than the effects of depleting anti-LAG-3 antibodies since they do not cause the destruction of activated T cells. It will be appreciated that the length of time for which an antibody of the invention is effective will depend on the plasma half-life of the antibody.

Optionally a binding molecule of the invention is an agonist of LAG-3 that inhibits antigen-induced CD4$^+$ and/or CD8$^+$ T cell proliferation, and/or antigen-induced CD4$^+$ and/or CD8$^+$ T cell activation.

In some embodiments, a binding molecule of the invention inhibits antigen-induced CD4$^+$ T cell proliferation, and antigen-induced CD8$^+$ T cell proliferation. In particular embodiments, the binding molecule inhibits antigen-induced CD8$^+$ T cell proliferation more than antigen-induced CD4$^+$ T cell proliferation.

Inhibition of antigen-induced CD4$^+$ and/or CD8$^+$ T cell proliferation may be determined by any suitable method known to the skilled person. An example of a suitable method is by measuring the proliferation of CD4$^+$ and/or CD8$^+$ T cells induced by antigenic peptides in the presence of the antibody or fragment, compared with the corresponding proliferation in the presence of a negative control antibody of the same isotype. The CD4$^+$ and CD8$^+$ T cells may be present, for example, in a sample of peripheral blood mononuclear cells (PBMCs) obtained from a healthy donor. Proliferation of the cells may be induced by any suitable antigenic peptides, such as a pool of peptides covering the sequence of CMV pp35. Proliferation of the cells may be measured by labelling the cells, for example with a fluorescent cell staining dye, such as carboxyfluorescein succinimidyl ester (CFSE). An example of a method for determining inhibition of antigen-induced CD4$^+$ and/or CD8$^+$ T cell proliferation is described in more detail in Example 10 of WO 2017/037203.

The percentage inhibition of antigen-induced CD4$^+$ and/or CD8$^+$ T cell proliferation may be determined as a percentage inhibition of the proliferation index (PI), calculated as the sum of: the percentage of CD4$^+$ and/or CD8$^+$ T cells under each division peak (assessed by FACS), multiplied by the division number, as described in more detail in Example 10 of WO 2017/037203.

In some embodiments, a binding molecule of the invention inhibits antigen-induced CD4$^+$ T cell proliferation by at least 10%, 20%, 30%, 40%, 50%, 60%, 70%, 80%, or 90%, compared to antigen-induced CD4$^+$ T cell proliferation in the absence of the binding molecule.

In some embodiments, a binding molecule of the invention inhibits antigen-induced CD8$^+$ T cell proliferation by at least 10%, 20%, 30%, 40%, 50%, 60%, 70%, 80%, or 90%, compared to antigen-induced CD8$^+$ T cell proliferation in the absence of the binding molecule.

In some embodiments, a binding molecule of the invention inhibits antigen-induced CD4$^+$ T cell proliferation, and antigen-induced CD8$^+$ T cell proliferation, each by at least 10%, 20%, 30%, 40%, 50%, 60%, 70%, 80%, or 90%, compared to antigen-induced CD4$^+$ T cell proliferation, and antigen-induced CD8$^+$ T cell proliferation, respectively, in the absence of the binding molecule.

In one embodiment, a binding molecule of the invention inhibits antigen-induced CD4$^+$ T cell proliferation by at least 20% compared to antigen-induced CD4$^+$ T cell proliferation in the absence of the binding molecule, and inhibits antigen-induced CD8$^+$ T cell proliferation by at least 30% compared to and antigen-induced CD8$^+$ T cell proliferation in the absence of the binding molecule.

Inhibition of antigen-induced CD4+ and/or CD8+ T cell proliferation by a binding molecule of the invention may be compared to antigen-induced CD4+ and/or CD8+ T cell proliferation in the presence of a negative control molecule (for example, where the binding molecule is an antibody, an antibody of the same isotype).

In some embodiments, a binding molecule of the invention inhibits antigen-induced CD8$^+$ T cell proliferation by at least 10%, 20%, 30%, 40%, 50%, 60%, 70%, 80%, or 90%, more than the binding molecule inhibits antigen-induced CD4$^+$ T cell proliferation.

According to some embodiments, the inhibition of antigen-induced CD8$^+$ T cell proliferation is LAG-3 dependent and IL-2 independent.

According to some embodiments, a binding molecule of the invention inhibits antigen-induced CD4$^+$ and/or CD8$^+$ T cell activation. In certain embodiments, a binding molecule of the invention inhibits antigen-induced CD4$^+$ and/or CD8$^+$ T cell proliferation and antigen-induced CD4$^+$ and/or CD8$^+$ T cell activation.

In particular, a binding molecule of the invention may bind to LAG-3 and inhibit antigen-induced CD4$^+$ and/or CD8$^+$ T cell activation. In certain embodiments, a binding molecule of the invention binds to LAG-3 and inhibits antigen-induced CD4$^+$ T cell activation and antigen-induced CD8$^+$ T cell activation.

Inhibition of activation of CD4$^+$ and/or CD8$^+$ T cells may be determined by any suitable method known to the skilled person. An example of a suitable method is by measuring the effect of the binding molecule on CD4$^+$ and/or CD8$^+$ T cell activation marker expression, or T cell activation marker secretion. For example, CD8$^+$ T cell activation may be measured by measuring the expression of CD25, as an activation marker, on CD8$^+$ T cells induced by antigenic peptides in the presence of the binding molecule, compared with the corresponding CD25 expression in the presence of a negative control molecule (for example, where the binding molecule is an antibody, an antibody of the same isotype). Alternatively, T cell activation may be measured by measuring the secretion of IFN-γ in cell supernatant of T cells induced by antigenic peptides in the presence of the binding molecule, compared with the corresponding secretion in the presence of a negative control molecule (for example, wherein the binding molecule is an antibody, an antibody of the same isotype). The T cells may be present, for example, in a sample of PBMCs obtained from a healthy donor. Activation of the cells may be induced by any suitable antigenic peptides, such as a pool of peptides covering the sequence of CMV pp35. An example of a method for determining inhibition of antigen-induced T cell activation by measuring T cell activation marker secretion is described in more detail in Example 15 of WO 2017/037203. An example of a method for determining inhibition of antigen-induced CD8$^+$ T cell activation by measuring CD8$^+$ T cell activation marker expression is described in more detail in Example 16 of WO 2017/037203.

In some embodiments, a binding molecule of the invention inhibits antigen-induced CD4$^+$ and/or CD8$^+$ T cell activation by at least 10%, 20%, 30%, 40%, 50%, 60%, 70%, 80%, or 90%, compared to antigen-induced CD4$^+$ and/or CD8$^+$ T cell activation in the absence of the binding molecule.

Inhibition of antigen-induced CD4+ and/or CD8+ T cell activation by a binding molecule of the invention may be compared to antigen-induced CD4+ and/or CD8+ T cell activation in the presence of a negative control molecule (for example, where the binding molecule is an antibody, an antibody of the same isotype).

According to the invention there is also provided use of an agonist of LAG-3 to inhibit expression of a NFAT-regulated gene in a LAG-3 positive T cell.

Inhibition of NFAT-regulated gene expression may be *in vivo* or *in vitro.*

Optionally the NFAT-regulated gene is a gene encoding a cytokine or a chemokine. Optionally the NFAT-regulated gene is a gene encoding IL-2.

Optionally the agonist of LAG-3 is a binding molecule of the invention. Optionally the agonist of LAG-3 is IMP761, or an antigen-binding fragment thereof.

According to some embodiments, a binding molecule of the invention inhibits binding of IMP321 to MHC class II-positive cells.

IMP321 (also referred to as "LAG-3Ig" below) is a recombinant soluble human LAG-3Ig fusion protein. The fusion protein is obtained as a 200-kDa dimer produced in Chinese hamster ovary (CHO) cells transfected with a plasmid encoding for the extracellular domain of human LAG-3 fused to the human IgG1 Fc. The sequence of IMP321 is provided in SEQ ID NO:17 of US Patent Application No. 2011/0008331.

Binding of IMP321 to MHC class II-positive cells may be determined by measuring binding of an IMP321-label conjugate (for example an IMP321-Alex 488 conjugate) to Raji cells (these are MHC class II-positive B cells), for example as described in Example 8 of WO 2017/037203.

In some embodiments, a binding molecule of the invention inhibits binding of IMP321 to MHC class II-positive cells by at least 10%, at least 20%, at least 30%, at least 40%, at least 50%, at least 60%, at least 70%, at least 80%, or at least 90% compared to the binding of IMP321 to MHC class II-positive cells in the absence of the binding molecule.

In some embodiments, a binding molecule of the invention inhibits binding of IMP321 to MHC class II-positive cells by at least 30%, compared to the binding of IMP321 to MHC class II-positive cells in the absence of the binding molecule, wherein the ratio of the concentration of the binding molecule to IMP321 is 0.1:1.

In some embodiments, a binding molecule of the invention inhibits binding of IMP321 to MHC class II-positive cells by at least 80%, compared to the binding of IMP321 to MHC class II-positive cells in the absence of the binding molecule, wherein the ratio of the concentration of the binding molecule to IMP321 is 0.3:1 or 1:1.

In some embodiments, a binding molecule of the invention inhibits IMP321-induced monocyte activation.

IMP321 is able to activate cells of the human monocytic cell line THP-1. Activation of THP-1 cells can be determined by the level of secretion of chemokine ligand 4 (CCL4, also known as Macrophage inflammatory protein-1β, MIP-1β). Pre-incubation of a binding molecule of the invention with IMP321 prior to incubation of the mixture with THP-1 cells can be used to determine whether the binding molecule inhibits IMP321-induced monocyte activation. A method for determining inhibition of IMP321-induced monocyte activation is described in more detail in Example 9 of WO 2017/037203.

In some embodiments, a binding molecule of the invention inhibits IMP321-induced monocyte activation by at least 10%, at least 20%, at least 30%, at least 40%, at least 50%, at least 60%, at least 70%, at least 80%, or at least 90% compared to the amount of IMP321-induced monocyte activation in the absence of the binding molecule.

In some embodiments, a binding molecule of the invention inhibits IMP321-induced monocyte activation by at least 70% compared to the amount of IMP321-induced monocyte activation in the absence of the binding molecule, wherein the ratio of the concentration of the antibody, or fragment, to IMP321 is 1:1.

A binding molecule of the invention does not bind to amino acid residues of the 30 amino acid extra-loop sequence (SEQ ID NO:18) of the first N-terminal D1 domain of human LAG-3 protein.

A binding molecule of the invention may inhibit binding of LAG-3 to MHC class II molecules *in vivo*. In particular, a binding molecule of the invention may antagonise the MHC class II-activating signal into antigen-presenting cells (APCs). Thus, a binding molecule of the invention may inhibit LAG-3-induced APC activation, for example dendritic cell activation, for example LAG-3-induced monocyte or macrophage activation.

In some embodiments, a binding molecule of the invention inhibits binding of LAG-3 to MHC class II-positive cells by at least 10%, at least 20%, at least 30%, at least 40%, at least 50%, at least 60%, at least 70%, at least 80%, or at least 90% compared to the binding of LAG-3 to MHC class II-positive cells in the absence of the binding molecule.

In some embodiments, a binding molecule of the invention inhibits LAG-3-induced APC activation by at least 10%, at least 20%, at least 30%, at least 40%, at least 50%, at least 60%, at least 70%, at least 80%, or at least 90% compared to the amount of LAG-3-induced APC activation in the absence of the binding molecule.

For nucleotide and amino acid sequences, the term "identical" or "identity" indicates the degree of identity between two nucleic acid or two amino acid sequences when optimally aligned and compared with appropriate insertions or deletions.

The percent identity between two sequences is a function of the number of identical positions shared by the sequences (i.e., % identity = number of identical positions/total number of positions multiplied by 100), taking into account the number of gaps, and the length of each gap, which need to be introduced for optimal alignment of the two sequences. The comparison of sequences and determination of percent identity between two sequences can be accomplished using a mathematical algorithm, as described below.

Percent identity between a query nucleic acid sequence and a subject nucleic acid sequence is the "Identities" value, expressed as a percentage, which is calculated by the BLASTN algorithm when a subject nucleic acid sequence has 100% query coverage with a query nucleic acid sequence after a pair-wise BLASTN alignment is performed. Such pair-wise BLASTN alignments between a query nucleic acid sequence and a subject nucleic acid sequence are performed by using the default settings of the BLASTN algorithm available on the National Center for Biotechnology Institute's website with the filter for low complexity regions turned off. Importantly, a query nucleic acid sequence may be described by a nucleic acid sequence identified in one or more claims herein.

Percent identity between a query amino acid sequence and a subject amino acid sequence is the "Identities" value, expressed as a percentage, which is calculated by the BLASTP algorithm when a subject amino acid sequence has 100% query coverage with a query amino acid sequence after a pair-wise BLASTP alignment is performed. Such pair-wise BLASTP alignments between a query amino acid sequence and a subject amino acid sequence are performed by using the default settings of the BLASTP algorithm available on the National Center for Biotechnology Institute's website with the filter for low complexity regions turned off. Importantly, a query amino acid sequence may be described by an amino acid sequence identified in one or more claims herein.

Compositions

The present disclosure provides a composition comprising a subject binding molecule. A subject binding molecule composition can comprise, in addition to a subject binding molecule, one or more of: a salt, e.g., NaCl, $MgCl_2$, KCl, $MgSO_4$, etc.; a buffering agent, e.g., a phosphate buffer, a citrate buffer, a Tris buffer, N-(2-Hydroxyethyl)piperazine-N'-(2-ethanesulfonic acid) (HEPES), 2-(N-Morpholino)ethanesulfonic acid (MES), 2-(N-Morpholino)ethanesulfonic acid sodium salt (MES), 3-(N-Morpholino)propanesulfonic acid (MOPS), N-tris[Hydroxymethyl]methyl-3-aminopropanesulfonic acid (TAPS), etc.; a solubilizing agent; a detergent, e.g., a non-ionic detergent such as Tween-20, etc.; a protease inhibitor; glycerol; and the like.

Pharmaceutical Compositions

There is also provided according to the invention a pharmaceutical composition comprising a binding molecule of the invention, and a pharmaceutically acceptable carrier, excipient, or diluent.

The present disclosure provides compositions, including pharmaceutical compositions comprising a subject binding molecule. In general, a pharmaceutical composition, also referred to herein as a formulation, comprises an effective amount of a subject binding molecule. An "effective amount" means a dosage sufficient to produce a desired result, e.g., reduction in an adverse symptom associated with an immune disorder, amelioration of a symptom of an immune disorder, slowing progression of an immune disorder, etc. Generally, the desired result is at least a reduction in a symptom of an immune disorder, as compared to a control.

Formulations

In the subject methods, a subject binding molecule can be administered to the host using any convenient means capable of resulting in the desired therapeutic effect or diagnostic effect. Thus, the agent can be incorporated into a variety of formulations for therapeutic administration. More particularly, a subject binding molecule can be formulated into pharmaceutical compositions by combination with appropriate, pharmaceutically acceptable carriers, pharmaceutically acceptable diluents, or other pharmaceutically acceptable excipients and can be formulated into preparations in solid, semi-solid, liquid or gaseous forms, such as tablets, capsules, powders, granules, ointments, solutions, suppositories, injections, inhalants and aerosols. In some embodiments, a pharmaceutical composition comprises a subject binding molecule and a pharmaceutically acceptable excipient.

In pharmaceutical dosage forms, a subject binding molecule can be administered in the form of their pharmaceutically acceptable salts, or they can also be used alone or in appropriate association, as well as in combination, with other pharmaceutically active compounds. The following methods and excipients are merely exemplary and are in no way limiting.

For oral preparations, a subject binding molecule can be used alone or in combination with appropriate additives to make tablets, powders, granules or capsules, for example, with conventional additives, such as lactose, mannitol, corn starch or potato starch; with binders, such as crystalline cellulose, cellulose derivatives, acacia, corn starch or gelatins; with disintegrators, such as corn starch, potato starch or sodium carboxymethylcellulose; with lubricants, such as talc or magnesium stearate; and if desired, with diluents, buffering agents, moistening agents, preservatives and flavoring agents.

A subject binding molecule can be formulated into preparations for injection by dissolving, suspending or emulsifying the antibody in an aqueous or nonaqueous solvent, such as vegetable or other similar oils, propylene glycol, synthetic aliphatic acid glycerides, injectable organic esters (e.g., ethyl oleate), esters of higher aliphatic acids or propylene glycol; and if desired, with conventional additives such as solubilizers, isotonic agents, suspending agents, emulsifying agents, stabilizers and preservatives. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Furthermore, a pharmaceutical composition of the present disclosure can comprise further agents such as dopamine or psychopharmacologic drugs, depending on the intended use of the pharmaceutical composition.

Pharmaceutical compositions comprising a subject binding molecule are prepared by mixing a subject binding molecule having the desired degree of purity with optional physiologically acceptable carriers, other excipients, stabilizers, surfactants, buffers and/or tonicity agents. Acceptable carriers, other excipients and/or stabilizers are nontoxic to recipients at the dosages and concentrations employed, and include buffers such as phosphate, citrate, and other organic acids; antioxidants including ascorbic acid, glutathione, cysteine, methionine and citric acid; preservatives (such as ethanol, benzyl alcohol, phenol, m-cresol, p-chlor-m-cresol, methyl or propyl parabens, benzalkonium chloride, or combinations thereof); amino acids such as arginine, glycine, ornithine, lysine, histidine, glutamic acid, aspartic acid, isoleucine, leucine, alanine, phenylalanine, tyrosine, tryptophan, methionine, serine, proline and combinations thereof; monosaccharides, disaccharides and other carbohydrates; low molecular weight (less than about 10 residues) polypeptides; proteins, such as gelatin or serum albumin; chelating agents such as EDTA; sugars such as trehalose, sucrose, lactose, glucose, mannose, maltose, galactose, fructose, sorbose, raffinose, glucosamine, N-methylglucosamine, galactosamine, and neuraminic acid; and/or non-ionic surfactants such as Tween, Brij Pluronics, Triton-X, or polyethylene glycol (PEG).

The pharmaceutical composition can be in a liquid form, a lyophilized form or a liquid form reconstituted from a lyophilized form, wherein the lyophilized preparation is to be reconstituted with a sterile solution prior to administration. The standard procedure for reconstituting a lyophilized composition is to add back a volume of pure water (typically equivalent to the volume removed during lyophilization); however solutions comprising antibacterial agents can be used for the production of pharmaceutical compositions for parenteral administration; see also Chen (1992) Drug Dev Ind Pharm 18, 1311-54.

Exemplary binding molecule concentrations in a subject pharmaceutical composition can range from about 1 mg/mL to about 200 mg/mL or from about 50 mg/mL to about 200 mg/mL, or from about 150 mg/mL to about 200 mg/mL.

An aqueous formulation of the binding molecule can be prepared in a pH-buffered solution, e.g., at pH ranging from about 4.0 to about 7.0, or from about 5.0 to about 6.0, or alternatively about 5.5. Examples of buffers that are suitable for a pH within this range include phosphate-, histidine-, citrate-, succinate-, acetate-buffers and other organic acid buffers. The buffer concentration can be from about 1 mM to about 100 mM, or from about 5 mM to about 50 mM, depending, e.g., on the buffer and the desired tonicity of the formulation.

A tonicity agent can be included in the binding molecule formulation to modulate the tonicity of the formulation. Exemplary tonicity agents include sodium chloride, potassium chloride, glycerin and any component from the group of amino acids, sugars as well as combinations thereof. In some embodiments, the aqueous formulation is isotonic, although hypertonic or hypotonic solutions can be suitable. The term "isotonic" denotes a solution having the same tonicity as some other solution with which it is compared, such as a physiological salt solution or serum. Tonicity agents can be used in an amount of about 5 mM to about 350 mM, e.g., in an amount of 100 mM to 350 nM.

A surfactant can also be added to the binding molecule formulation to reduce aggregation of the formulated binding molecule and/or minimize the formation of particulates in the formulation and/or reduce adsorption. Exemplary surfactants include polyoxyethylensorbitan fatty acid esters (Tween), polyoxyethylene alkyl ethers (Brij), alkylphenylpolyoxyethylene ethers (Triton-X), polyoxyethylene-polyoxypropylene copolymer (Poloxamer, Pluronic), and sodium dodecyl sulfate (SDS). Examples of suitable polyoxyethylenesorbitan-fatty acid esters are polysorbate 20, (sold under the trademark Tween 20™) and polysorbate 80 (sold under the trademark Tween 80™). Examples of suitable polyethylene-polypropylene copolymers are those sold under the names Pluronic® F68 or Poloxamer 188™. Examples of suitable Polyoxyethylene alkyl ethers are those sold under the trademark Brij™. Exemplary concentrations of surfactant can range from about 0.001% to about 1% w/v.

A lyoprotectant can also be added in order to protect the labile active ingredient (e.g. a protein) against destabilizing conditions during the lyophilization process. For example, known lyoprotectants include sugars (including glucose and sucrose); polyols (including mannitol, sorbitol and glycerol); and amino acids (including alanine, glycine and glutamic acid). Lyoprotectants can be included in an amount of about 10 mM to 500 nM.

In some embodiments, a subject formulation includes a subject binding molecule, and one or more of the above-identified agents (e.g., a surfactant, a buffer, a stabilizer, a tonicity agent) and is essentially free of one or more preservatives, such as ethanol, benzyl alcohol, phenol, m-cresol, p-chlor-m-cresol, methyl or propyl parabens, benzalkonium chloride, and combinations thereof. In other embodiments, a preservative is included in the formulation, e.g., at concentrations ranging from about 0.001 to about 2% (w/v).

For example, a subject formulation can be a liquid or lyophilized formulation suitable for parenteral administration, and can comprise: about 1 mg/mL to about 200 mg/mL of a subject antibody; about 0.001 % to about 1 % of at least one surfactant; about 1 mM to about 100 mM of a buffer; optionally about 10 mM to about 500 mM of a stabilizer; and about 5 mM to about 305 mM of a tonicity agent; and has a pH of about 4.0 to about 7.0.

As another example, a subject parenteral formulation is a liquid or lyophilized formulation comprising: about 1 mg/mL to about 200 mg/mL of a subject antibody; 0.04% Tween 20 w/v; 20 mM L-histidine; and 250 mM Sucrose; and has a pH of 5.5.

As another example, a subject parenteral formulation comprises a lyophilized formulation comprising: 1) 15 mg/mL of a subject antibody; 0.04% Tween 20 w/v; 20 mM L-histidine; and 250 mM sucrose; and has a pH of 5.5; or 2) 75 mg/mL of a subject antibody; 0.04% Tween 20 w/v; 20 mM L-histidine; and 250 mM sucrose; and has a pH of 5.5;or 3) 75 mg/mL of a subject antibody; 0.02% Tween 20 w/v; 20 mM L-histidine; and 250 mM sucrose; and has a pH of 5.5; or 4) 75 mg/mL of a subject antibody; 0.04% Tween 20 w/v; 20 mM L-histidine; and 250 mM trehalose; and has a pH of 5.5; or 5) 75 mg/mL of a subject antibody; 0.02% Tween 20 w/v; 20 mM L-histidine; and 250 mM trehalose; and has a pH of 5.5.

As another example, a subject parenteral formulation is a liquid formulation comprising:1) 7.5 mg/mL of a subject antibody; 0.02% Tween 20 w/v; 120 mM L-histidine; and 250 125 mM sucrose; and has a pH of 5.5; or 2) 37.5 mg/mL of a subject antibody; 0.02% Tween 20 w/v; 10 mM L-histidine; and 125 mM sucrose; and has a pH of 5.5; or 3) 37.5 mg/mL of a subject antibody; 0.01% Tween 20 w/v; 10 mM L-histidine; and 125 mM sucrose; and has a pH of 5.5; or 4) 37.5 mg/mL of a subject antibody; 0.02% Tween 20 w/v; 10 mM L-histidine; 125 mM trehalose; and has a pH of 5.5; or 5) 37.5 mg/mL of a subject antibody; 0.01% Tween 20 w/v; 10 mM L-histidine; and 125 mM trehalose; and has a pH of 5.5; or 6) 5 mg/mL of a subject antibody; 0.02% Tween 20 w/v; 20 mM L-histidine; and 250 mM trehalose; and has a pH of 5.5; or 7) 75 mg/mL of a subject antibody; 0.02% Tween 20 w/v; 20 mM L-histidine; and 250 mM mannitol; and has a pH of 5.5; or 8) 75 mg/mL of a subject antibody; 0.02% Tween 20 w/v; 20 mM L histidine; and 140 mM sodium chloride; and has a pH of 5.5;or 9) 150 mg/mL of a subject antibody; 0.02% Tween 20 w/v; 20 mM L-histidine; and 250 mM trehalose; and has a pH of 5.5; or 10) 150 mg/mL of a subject antibody; 0.02% Tween 20 w/v; 20 mM L-histidine; and 250 mM mannitol; and has a pH of 5.5; or 11) 150 mg/mL of a subject antibody; 0.02% Tween 20 w/v; 20 mM L-histidine; and 140 mM sodium chloride; and has a pH of 5.5; or 12) 10 mg/mL of a subject antibody; 0.01% Tween 20 w/v; 20 mM L-histidine; and 40 mM sodium chloride; and has a pH of 5.5.

A subject binding molecule can be utilized in aerosol formulation to be administered via inhalation. A subject binding molecule can be formulated into pressurized acceptable propellants such as dichlorodifluoromethane, propane, nitrogen and the like. Aerosol formulations such as nasal spray formulations include purified aqueous or other solutions of the active agent with preservative agents and isotonic agents. Such formulations are adjusted to a pH and isotonic state compatible with the nasal mucous membranes.

Furthermore, a subject binding molecule can be made into suppositories by mixing with a variety of bases such as emulsifying bases or water-soluble bases. A subject binding molecule can be administered rectally via a suppository. The suppository can include vehicles such as cocoa butter, carbowaxes and polyethylene glycols, which melt at body temperature, yet are solidified at room temperature.

Unit dosage forms for oral or rectal administration such as syrups, elixirs, and suspensions can be provided wherein each dosage unit, for example, teaspoonful, tablespoonful, tablet or suppository, contains a predetermined amount of the composition. Similarly, unit dosage forms for injection or intravenous administration can comprise a subject binding molecule in a composition as a solution in sterile water, normal saline or another pharmaceutically acceptable carrier.

The term "unit dosage form," as used herein, refers to physically discrete units suitable as unitary dosages for human and animal subjects, each unit containing a predetermined quantity of a binding molecule of the present disclosure, calculated in an amount sufficient to produce the desired effect in association with a pharmaceutically acceptable diluent, carrier or vehicle. The specifications for a subject binding molecule can depend on the particular binding molecule employed and the effect to be achieved, and the pharmacodynamics associated with each binding molecule in the host.

Other modes of administration will also find use with a method of the present disclosure. For instance, a subject binding molecule can be formulated in suppositories and, in some cases, aerosol and intranasal compositions. For suppositories, the vehicle composition will include traditional binders and carriers such as, polyalkylene glycols, or triglycerides. Such suppositories can be formed from mixtures containing the active ingredient in the range of about 0.5% to about 10% (w/w), e.g., about 1% to about 2%.

Intranasal formulations will usually include vehicles that neither cause irritation to the nasal mucosa nor significantly disturb ciliary function. Diluents such as water, aqueous saline or other known substances can be employed. The nasal formulations can also contain preservatives such as, but not limited to, chlorobutanol and benzalkonium chloride. A surfactant can be present to enhance absorption of the subject binding molecule by the nasal mucosa.

A subject binding molecule can be administered as an injectable formulation. Typically, injectable compositions are prepared as liquid solutions or suspensions; solid forms suitable for solution in, or suspension in, liquid vehicles prior to injection can also be prepared. The preparation can also be emulsified or the binding molecule encapsulated in liposome vehicles.

Suitable excipient vehicles are, for example, water, saline, dextrose, glycerol, ethanol, or the like, and combinations thereof. In addition, if desired, the vehicle can contain minor amounts of auxiliary substances such as wetting or emulsifying agents or pH buffering agents. Actual methods of preparing such dosage forms are known, or will be apparent, to those skilled in the art. See, e.g., Remington's Pharmaceutical Sciences, Mack Publishing Company, Easton, Pennsylvania, 17th edition, 1985. The composition or formulation to be administered will, in any event, contain a quantity of a subject binding molecule adequate to achieve the desired state in the subject being treated.

The pharmaceutically acceptable excipients, such as vehicles, adjuvants, carriers or diluents, are readily available to the public. Moreover, pharmaceutically acceptable auxiliary substances, such as pH adjusting and buffering agents, tonicity adjusting agents, stabilizers, wetting agents and the like, are readily available to the public.

In some embodiments, a subject binding molecule is formulated in a controlled release formulation. Sustained-release preparations can be prepared using methods well known in the art. Suitable examples of sustained-release preparations include semipermeable matrices of solid hydrophobic polymers containing the antibody in which the matrices are in the form of shaped articles, e.g. films or microcapsules. Examples of sustained-release matrices include polyesters, copolymers of L-glutamic acid and ethyl-L-glutamate, nondegradable ethylene-vinyl acetate, hydrogels, polylactides, degradable lactic acid-glycolic acid copolymers and poly-D-(-)-3-hydroxybutyric acid. Possible loss of biological activity and possible changes in immunogenicity of binding molecules comprised in sustained-release preparations can be prevented by using appropriate additives, by controlling moisture content and by developing specific polymer matrix compositions.

Controlled release within the scope of the present disclosure can be taken to mean any one of a number of extended release dosage forms. The following terms can be considered to be substantially equivalent to controlled release, for the purposes of the present disclosure: continuous release, controlled release, delayed release, depot, extended release, gradual release, immediate release, long-term release, programmed release, prolonged release, proportionate release, protracted release, repository, retard, slow release, spaced release, sustained release, time coat, timed release, delayed action, extended action, layered-time action, long acting, prolonged action, repeated action, slowing acting, sustained action, and sustained-action medications. Further discussions of these terms can be found in Lesczek Krowczynski, Extended-Release Dosage Forms, 1987 (CRC Press, Inc.).

The various controlled release technologies cover a very broad spectrum of drug dosage forms. Controlled release technologies include, but are not limited to physical systems and chemical systems.

Physical systems include, but are not limited to, reservoir systems with rate-controlling membranes, such as microencapsulation, macroencapsulation, and membrane systems; reservoir systems without rate-controlling membranes, such as hollow fibers, ultra microporous cellulose triacetate, and porous polymeric substrates and foams; monolithic systems, including those systems physically dissolved in non-porous, polymeric, or elastomeric matrices (e.g., nonerodible, erodible, environmental agent ingression, and degradable), and materials physically dispersed in non-porous, polymeric, or elastomeric matrices (e.g., nonerodible, erodible, environmental agent ingression, and degradable); laminated structures, including reservoir layers chemically similar or dissimilar to outer control layers; and other physical methods, such as osmotic pumps, or adsorption onto ion-exchange resins.

Chemical systems include, but are not limited to, chemical erosion of polymer matrices (e.g., heterogeneous, or homogeneous erosion), or biological erosion of a polymer matrix (e.g., heterogeneous, or homogeneous). Additional discussion of categories of systems for controlled release can be found in Agis F. Kydonieus, Controlled Release Technologies: Methods, Theory and Applications, 1980 (CRC Press, Inc.).

There are a number of controlled release drug formulations that are developed for oral administration. These include, but are not limited to, osmotic pressure-controlled gastrointestinal delivery systems; hydrodynamic pressure-controlled gastrointestinal delivery systems; membrane permeation-controlled gastrointestinal delivery systems, which include microporous membrane permeation-controlled gastrointestinal delivery devices; gastric fluid-resistant intestine targeted controlled-release gastrointestinal delivery devices; gel diffusion-controlled gastrointestinal delivery systems; and ion-exchange-controlled gastrointestinal delivery systems, which include cationic and anionic drugs. Additional information regarding controlled release drug delivery systems can be found in Yie W. Chien, Novel Drug Delivery Systems, 1992 (Marcel Dekker, Inc.).

Dosages

A suitable dosage can be determined by an attending physician or other qualified medical personnel, based on various clinical factors. As is well known in the medical arts, dosages for any one patient depend upon many factors, including the patient's size, body surface area, age, the particular compound to be administered, sex of the patient, time, and route of administration, general health, and other drugs being administered concurrently. A subject binding molecule can be administered in amounts from 1 ng/kg body weight to 20 mg/kg body weight per dose, for example from 0.001 to 10, 0.01 to 10, 0.1 to 10, 1 to 10, 0.001 to 1, 0.01 to 1, 0.1 to 1, 0.05 to 5, 0.05 to 0.5, or 0.5 to 5 mg/kg body weight. However, doses below or above this exemplary range are envisioned, especially considering the aforementioned factors. If the regimen is a continuous infusion, it may be in the range of 1 $\mu$g to 10 mg/kg of body weight per minute.

In some embodiments, a dose of a subject binding molecule is in the range of 0.001 $\mu$g to 100mg, for example 0.001 $\mu$g to 10mg, 0.001 $\mu$g to 1mg, 0.001 $\mu$g to 0.1 mg, 0.01 $\mu$g to 10mg, 0.1 $\mu$g to 10mg, 0.1 $\mu$g to 1 mg, or 0.1 $\mu$g to 0.1 mg, or 0.01 to 100 mg, 0.01 to 10 mg, 0.01 to 1 mg, 0.01 to 0.1 mg, 0.1 to 100 mg, 0.1 to 10 mg, 0.1 to 1 mg.

In some embodiments, the dosage can range, for example from about 0.0001 to 100 mg/kg, or from about 0.01 to 5 mg/kg (e.g., 0.02 to 5 mg/kg, 0.25 to 5 mg/kg, 0.5 to 5 mg/kg, 0.75 to 5 mg/kg, 1 to 5 mg/kg, 2 to 5 mg/kg, etc.) body weight. For example, dosages can be 0.1, 1, or 10 mg/kg body weight or within the range of 0.01-10 mg/kg, or at least 0.1 mg/kg.

In particular embodiments, a dose of a binding molecule of the invention is up to 0.5 mg/kg body weight, for example in the range of 0.0001 to 0.5 mg/kg, 0.001 to 0.5 mg/kg, 0.01 to 0.5 mg/kg, 0.1 to 0.5 mg/kg, 0.0001 to 0.1 mg/kg, 0.001 to 0.1 mg/kg, 0.01 to 0.1 mg/kg body weight.

In some embodiments, a subject binding molecule is administered in an amount that provides for a peak serum concentration of from about 0.001 $\mu$g/ml to about 1 mg/ml, for example 0.0001 $\mu$g/ml to 1 $\mu$g/ml, 0.001 $\mu$g/ml to 1 $\mu$g/ml, 0.001 $\mu$g/ml to 0.1 $\mu$g/ml, 0.01 to 1, or 0.01 to 0.1 $\mu$g/ml, or from about 0.005 $\mu$g/ml to about 1 $\mu$g/ml, or from about 0.1 $\mu$g/ml to about 1 $\mu$g/ml, or from about 1 $\mu$g/ml to about 2.5 $\mu$g/ml, from about 2.5 $\mu$g/ml to about 5 $\mu$g/ml, from about 5 $\mu$g/ml to about 7.5 $\mu$g/ml, from about 7.5 $\mu$g/ml to about 10 $\mu$g/ml, from about 10 $\mu$g/ml to about 25 $\mu$g/ml, from about 25 $\mu$g/ml to about 50 $\mu$g/ml, from about 50 $\mu$g/ml to about 100 $\mu$g/ml, from about 100 $\mu$g/ml to about 250 $\mu$g/ml, from about 250 $\mu$g/ml to about 500 $\mu$g/ml, from about 500 $\mu$g/ml to about 750 $\mu$g/ml, or from about 750 $\mu$g/ml to about 1000 $\mu$g/ml. In some embodiments, a subject binding molecule is administered in an amount that provides for a peak serum concentration of greater than 1 mg/ml, e.g., from about 1 mg/ml to about 2 mg/ml, from about 2 mg/ml to about 5 mg/ml, or from about 5 mg/ml to about 10 mg/ml. In other embodiments, a binding molecule of the invention is administered in an amount that provides for a peak serum concentration of up to 1 $\mu$g/ml, for example in the range of 0.0001 $\mu$g/ml to 1 $\mu$g/ml, 0.001 $\mu$g/ml to 1 $\mu$g/ml, 0.01 $\mu$g/ml to 1 $\mu$g/ml, 0.1 to 1 $\mu$g/ml, 0.0001 $\mu$g/ml to 0.1 $\mu$g/ml, 0.001 $\mu$g/ml to 0.1 $\mu$g/ml, or 0.01 $\mu$g/ml to 0.1 $\mu$g/ml. Suitably such administration is by subcutaneous injection.

Individuals can be administered such doses daily, on alternative days, weekly or according to any other schedule determined by empirical analysis. An exemplary treatment entails administration in multiple dosages over a prolonged period, for example, of at least six months. Additional exemplary treatment regimens entail administration once per every two weeks or once a month or once every 3 to 6 months.

Exemplary dosage schedules include 0.01 to 1 mg/kg, 0.01 to 0.1 mg/kg, 0.1 to 1 mg/kg, 1 to 10 mg/kg or 15 mg/kg on consecutive days, 0.02 to 20 mg/kg, for example 0.2 mg/kg, 0.5 mg/kg, 1 mg/kg, 5 mg/kg, 10 mg/kg, or 20 mg/kg on alternate days, or 0.1 to 100 mg/kg, for example 1 mg/kg, 10 mg/kg, 20 mg/kg, 30 mg/kg, 40 mg/kg, 50 mg/kg, or 60 mg/kg weekly. In some methods, two or more binding molecule with different binding specificities are administered simultaneously, in which case the dosage of each binding molecule administered falls within the ranges indicated. Progress can be monitored by periodic assessment.

The number of CD4+ and/or CD8+ T cells expressing LAG-3 in a subject is relatively low. It is expected that a single administration of a binding molecule of the invention (in particular, a binding molecule of the invention that has a serum half-life of at least two weeks and that lacks significant CDC and ADCC activity, such as a human IgG isotype antibody (which lacks CDC and ADCC activity), or an antibody that comprises one or more mutations to reduce or abolish CDC and ADCC activity) may be effective at inhibiting antigen-induced CD4+ and/or CD8+ T cell proliferation for at least several weeks. In view of this, a suitable treatment regimen may comprise administration of a binding molecule of the invention (for example 0.01 to 1 mg/kg of the binding molecule) once every four, six, eight, or ten weeks, or once every two or three months. Such treatment may be provided over a period of at least six months, or at least one, two, three, four, or five years, or longer, for example throughout the course of a disease that is treated by the administration, or throughout the lifetime of the subject.

Those of skill will readily appreciate that dose levels and administration schedules can vary as a function of the specific binding molecule, the severity of the symptoms and the susceptibility of the subject to side effects. Preferred dosages and administration schedules for a given binding molecule are readily determinable by those of skill in the art by a variety of means.

Routes of administration

A subject binding molecule is administered to an individual using any available method and route suitable for drug delivery, including *in vivo* and *ex vivo* methods, as well as systemic and localized routes of administration.

Conventional and pharmaceutically acceptable routes of administration include intranasal, intramuscular, intratracheal, intrathecal, intracranial, subcutaneous, intradermal, topical, intravenous, intraperitoneal, intraarterial (e.g., via the carotid artery), spinal or brain delivery, rectal, nasal, oral, and other enteral and parenteral routes of administration. Routes of administration can be combined, if desired, or adjusted depending upon the binding molecule and/or the desired effect. A subject binding molecule composition can be administered in a single dose or in multiple doses. In some embodiments, a subject binding molecule composition is administered orally. In some embodiments, a subject binding molecule composition is administered via an inhalational route. In some embodiments, a subject binding molecule composition is administered intranasally. In some embodiments, a subject binding molecule composition is administered locally. In some embodiments, a subject binding molecule composition is administered intracranially. In some embodiments, a subject binding molecule composition is administered intravenously. In some embodiments, a subject binding molecule composition is administered intrathecally.

A binding molecule of the present disclosure can be administered to a host using any available conventional methods and routes suitable for delivery of conventional drugs, including systemic or localized routes. In general, routes of administration contemplated by the invention include, but are not necessarily limited to, enteral, parenteral, or inhalational routes.

Parenteral routes of administration other than inhalation administration include, but are not necessarily limited to, topical, transdermal, subcutaneous, intramuscular, intraorbital, intracapsular, intraspinal, intrasternal, intrathecal, and intravenous routes, *i.e.,* any route of administration other than through the alimentary canal. Parenteral administration can be carried to effect systemic or local delivery of a subject binding molecule. Where systemic delivery is desired, administration typically involves invasive or systemically absorbed topical or mucosal administration of pharmaceutical preparations.

A subject binding molecule can also be delivered to the subject by enteral administration. Enteral routes of administration include, but are not necessarily limited to, oral and rectal (e.g., using a suppository) delivery.

By "treatment" is meant at least an amelioration of the symptoms associated with the pathological condition afflicting the host, where amelioration is used in a broad sense to refer to at least a reduction in the magnitude of a parameter, e.g. symptom, associated with the pathological condition being treated, such as an immune disorder. As such, treatment also includes situations where the pathological condition, or at least symptoms associated therewith, are completely inhibited, *e.g.* prevented from happening, or stopped, *e.g.* terminated, such that the host no longer suffers from the pathological condition, or at least the symptoms that characterize the pathological condition.

In some embodiments, a subject binding molecule is administered by injection and/or delivery, e.g., to a site in a brain artery or directly into brain tissue. A subject binding molecule can also be administered directly to a target site e.g., by biolistic delivery to the target site.

A variety of hosts (wherein the term "host" is used interchangeably herein with the terms "subject," "individual," and "patient") are treatable according to the subject methods. Generally such hosts are "mammals" or "mammalian," where these terms are used broadly to describe organisms which are within the class mammalia, including the orders carnivore (e.g., cats), herbivores (e.g., cattle, horses, and sheep), omnivores (e.g., dogs, goats, and pigs), rodentia (*e.g.,* mice, guinea pigs, and rats), and primates (*e.g.,* humans, chimpanzees, and monkeys). In some embodiments, the host is an individual that has a complement system, such as a mammal, fish, or invertebrate. In some embodiments, the host is a complement system-containing mammal, fish, or invertebrate companion animal, agricultural animal, work animal, zoo animal, or lab animal. In some embodiments, the host is human.

The embodiments include compositions comprising a container suitable for containing a composition comprising a subject binding molecule for administration to an individual. For example, a subject binding molecule can be disposed within a container suitable for containing a pharmaceutical composition. The container can be, for example, a bottle (e.g., with a closure device, such as a cap), a blister pack (e.g., which can provide for enclosure of one or more doses per blister), a vial, flexible packaging (e.g., sealed Mylar or plastic bags), an ampule (for single doses in solution), a dropper, a syringe, thin film, a tube and the like. In some embodiments, a container, such as a sterile container, comprises a subject pharmaceutical composition. In some embodiments the container is a bottle or a syringe. In some embodiments the container is a bottle. In some embodiments the container is a syringe.

Kits with unit doses of a subject binding molecule, e.g. in oral or injectable doses, are provided. In such kits, in addition to the containers containing the unit doses will be an informational package insert describing the use and attendant benefits of the binding molecule in treating pathological condition of interest. Preferred compounds and unit doses are those described herein above.

Medical Use and Methods of Treatment

According to the invention there is also provided a binding molecule of the invention, or a pharmaceutical composition of the invention, for use as a medicament.

There is also provided according to the invention a binding molecule of the invention, or a pharmaceutical composition of the invention, for use in the treatment of a T cell-mediated immune disorder.

There is also provided according to the invention use of a binding molecule of the invention, or a pharmaceutical composition of the invention, in the manufacture of a medicament for the treatment of a T cell-mediated immune disorder.

The invention also provides a method of treating a T cell-mediated immune disorder, which comprises administering an effective amount of a binding molecule of the invention, or a pharmaceutical composition of the invention, to a subject in need of such treatment.

Optionally the T-cell-mediated immune disorder is an inflammatory disease, or an autoimmune disorder.

A binding molecule, or composition of the present invention may be utilised in any therapy where it is desired to increase the effects of LAG-3 in the subject.

The term "subject" includes any human or nonhuman animal. The term "nonhuman animal" includes all vertebrates, e.g., mammals and non-mammals, such as nonhuman primates, sheep, dogs, cats, cows, horses, chickens, amphibians, and reptiles, although mammals are preferred, such as non-human primates, sheep, dogs, cats, cows and horses.

The binding molecule or composition may be used in any therapy where it is desired to negatively regulate T cell proliferation and/or function.

There is also provided according to the invention a binding molecule of the invention, or a pharmaceutical composition of the invention, for use in the treatment of a disorder associated with proliferation or activity of CD4+ and/or CD8+ T cells in a subject, or a disorder associated with decreased expression and/or activity of LAG-3 in a subject.

There is also provided according to the invention use of a binding molecule of the invention, or a pharmaceutical composition of the invention, in the manufacture of a medicament for the treatment of a disorder associated with proliferation or activity of CD4+ and/or CD8+ T cells in a subject, or a disorder associated with decreased expression and/or activity of LAG-3 in a subject.

There is further provided according to the invention a method of treating a disorder associated with proliferation or activity of CD4+ and/or CD8+ T cells in a subject, or disorder associated with decreased expression and/or activity of LAG-3 in a subject, which comprises administering an effective amount of a binding molecule of the invention, or a pharmaceutical composition of the invention, to a subject in need of such treatment.

The disorder associated with proliferation or activity of CD4+ and/or CD8+ T cells may be an immune disorder, in particular a T-cell-mediated immune disorder, such as an inflammatory disease, or an autoimmune disorder.

The binding molecule or composition may be used to reduce the inflammatory process or to prevent the inflammatory process. In one embodiment there is provided an *in vivo* reduction of T cell proliferation or activation, in particular those involved in inappropriate inflammatory immune responses, for example recruited to the vicinity/location of such a response.

Reduction of T cell proliferation or activation, as employed herein, may be a reduction of 10, 20, 30, 40, 50, 60, 70, 80, 90 or more percent in comparison to before treatment or without treatment.

Advantageously, treatment with a binding molecule or composition according to the present invention may allow a reduction in the T cell proliferation or activation, without reducing the patient's general level of T cells (unactivated T cells). This may result in fewer side effects, and prevent T cell depletion in the patient.

The immune disorder, may, for example be selected from the group consisting of infections (viral, bacterial, fungal and parasitic), endotoxic shock associated with infection, sepsis, arthritis, rheumatoid arthritis, asthma, COPD, pelvic inflammatory disease, Alzheimer's Disease, inflammatory bowel disease, Crohn's disease, ulcerative colitis, Peyronie's Disease, coeliac disease, gallbladder disease, Pilonidal disease, peritonitis, psoriasis, vasculitis, surgical adhesions, stroke, Type I Diabetes, lyme disease, arthritis, meningoencephalitis, autoimmune uveitis, immune mediated inflammatory disorders of the central and peripheral nervous system such as multiple sclerosis, lupus (such as systemic lupus erythematosus) and Guillain-Barré syndrome, Atopic dermatitis, autoimmune hepatitis, fibrosing alveolitis, Grave's disease, IgA nephropathy, idiopathic thrombocytopenic purpura, Meniere's disease, pemphigus, primary biliary cirrhosis, sarcoidosis, scleroderma, Wegener's granulomatosis, other autoimmune disorders, pancreatitis, trauma (surgery), graft-versus-host disease, transplant rejection, heart disease including ischaemic diseases such as myocardial infarction as well as atherosclerosis, intravascular coagulation, bone resorption, osteoporosis, osteoarthritis, periodontitis and hypochlorhydia , or infertility related to lack of fetal-maternal tolerance.

A binding molecule or composition of the invention may be used in any therapy where it is desired to inhibit binding of LAG-3 to MHC class II molecules, to antagonise MHC class II-activating signal into antigen-presenting cells (APCs), or to inhibit LAG-3-induced APC activation.

There is also provided according to the invention a binding molecule of the invention, or a pharmaceutical composition of the invention, for use in the treatment of a disorder associated with activation of APCs in a subject.

There is also provided according to the invention use of a binding molecule of the invention, or a pharmaceutical composition of the invention, in the manufacture of a medicament for the treatment of a disorder associated with activation of APCs in a subject.

There is further provided according to the invention a method of treating a disorder associated with activation of APCs in a subject, which comprises administering an effective amount of a binding molecule of the invention, or a pharmaceutical composition of the invention, to a subject in need of such treatment.

As used herein, the terms "treatment", "treating", "treat" and the like, refer to obtaining a desired pharmacologic and/or physiologic effect. The effect can be prophylactic in terms of completely or partially preventing a disease or symptom thereof and/or can be therapeutic in terms of a partial or complete cure for a disease and/or adverse effect attributable to the disease. "Treatment" as used herein, covers any treatment of a disease in a mammal, particularly in a human, and includes: (a) preventing the disease from occurring in a subject which can be predisposed to the disease but has not yet been diagnosed as having it; (b) inhibiting the disease, i.e., arresting its development; and (c) relieving the disease, i.e., causing regression of the disease.

The terms "individual", "subject", "host", and "patient" used interchangeably herein includes any human or nonhuman animal. The term "nonhuman animal" includes all vertebrates, e.g., mammals and non-mammals, such as nonhuman primates, sheep, dogs, cats, cows, horses, chickens, amphibians, and reptiles, although mammals are preferred, such as non-human primates, sheep, dogs, cats, cows and horses.

A "therapeutically effective amount" or "efficacious amount" refers to the amount of a binding molecule of the invention that, when administered to a mammal or other subject for treating a disease, is sufficient to effect such treatment for the disease. The "therapeutically effective amount" will vary depending on the binding molecule, the disease and its severity and the age, weight, etc., of the subject to be treated.

All publications mentioned herein are incorporated herein by reference to disclose and describe the methods and/or materials in connection with which the publications are cited.

Aspects of the invention are defined in the numbered paragraphs below:

1. An isolated binding molecule, which is an agonist of LAG-3, and which inhibits TCR-mediated signal transduction in LAG-3 positive T cells through agonism of LAG-3.

2. A binding molecule according to paragraph 1, which inhibits a calcineurin-NFAT signalling pathway in LAG-3 positive T cells through agonism of LAG-3.

3. A binding molecule according to paragraph 1 or 2, which inhibits expression of one or more NFAT-regulated genes.

4. A binding molecule according to paragraph 3, wherein the one or more NFAT-regulated genes include genes encoding cytokines, receptors, or nuclear factors.

5. A binding molecule according to paragraph 3 or 4, wherein the one or more NFAT-regulated genes include genes encoding IL-2, IFN-y, IL-4, IL-6, IL-17, IL-21, IL-22, IL-23, CD25, PD-1, or TIM-3.

6. A binding molecule according to any of paragraphs 3 to 5, wherein the one or more NFAT-regulated genes includes a gene encoding IL-2.

7. A binding molecule according to any preceding paragraph, which inhibits antigen-induced CD4$^+$ and/or CD8$^+$ T-cell proliferation.

8. A binding molecule according to any preceding paragraph, which inhibits antigen-induced CD4$^+$ and/or CD8$^+$ T-cell activation.

9. A binding molecule according to any preceding paragraph, which binds specifically to a discontinuous epitope within the extracellular Ig superfamily domain D1 (SEQ ID NO:14) of a LAG-3 protein, wherein amino acid residues of the discontinuous epitope lie outside a 30 amino acid extra-loop sequence (SEQ ID NO:18) of the domain D1 of the LAG-3 protein.

10. An isolated binding molecule, which binds specifically to a discontinuous epitope within the extracellular Ig superfamily domain D1 (SEQ ID NO:14) of a LAG-3 protein, wherein amino acid residues of the discontinuous epitope lie outside a 30 amino acid extra-loop sequence (SEQ ID NO:18) of the domain D1 of the LAG-3 protein.

11. A binding molecule according to paragraph 10, which is an agonist of LAG-3.

12. A binding molecule according to any of paragraphs 9 to 11, wherein the discontinuous epitope comprises any of the following amino acid sequences:

TIPLQDLSLLRR          (SEQ ID NO:19);
GGLRSGRLPLQ           (SEQ ID NO:20);
QRGDFSLWLRPAR         (SEQ ID NO:21);
LRDRALSCRL            (SEQ ID NO:22).

13. A binding molecule according to any of paragraphs 9 to 12, wherein the discontinuous epitope comprises two or more of the following amino acid sequences:

TIPLQDLSLLRR          (SEQ ID NO:19);
GGLRSGRLPLQ           (SEQ ID NO:20);
QRGDFSLWLRPAR         (SEQ ID NO:21);
LRDRALSCRL            (SEQ ID NO:22).

14. A binding molecule according to any of paragraphs 9 to 13, wherein the discontinuous epitope comprises three or more of the following amino acid sequences:

TIPLQDLSLLRR          (SEQ ID NO:19);
GGLRSGRLPLQ           (SEQ ID NO:20);
QRGDFSLWLRPAR         (SEQ ID NO:21);
LRDRALSCRL            (SEQ ID NO:22).

15. A binding molecule according to any of paragraphs 9 to 14, wherein the discontinuous epitope comprises the following amino acid sequences:

TIPLQDLSLLRR          (SEQ ID NO:19);
GGLRSGRLPLQ           (SEQ ID NO:20);
QRGDFSLWLRPAR         (SEQ ID NO:21);
LRDRALSCRL            (SEQ ID NO:22).

and

16. A binding molecule according to any preceding paragraph, which binds to a human LAG-3 protein (or a human LAG-3Ig protein) with a dissociation constant ($K_D$) of no more than 100 pM, no more than 90 pM, no more than 80 pM, no more than 70 pM, no more than 60 pM, no more than 50 pM, no more than 40 pM, no more than 30 pM, or no more than 25 pM, for example as determined by Biacore analysis.

17. A binding molecule according to any preceding paragraph, wherein the binding molecule comprises an antibody, or an antigen-binding fragment thereof.

18. A binding molecule according to any preceding paragraph, wherein the binding molecule comprises one, two, or three complementarity determining regions (CDRs) of an antibody heavy chain variable (VH) region of IMP761 (VH CDRs1-3: SEQ ID NOs:1-3 or 7-9), and/or one, two, or three CDRs of an antibody light chain variable (VL) region of IMP761 (VL CDRs1-3: SEQ ID NOs:4-6 or 10-12).

19. A binding molecule according to any of paragraphs 1 to 17, wherein the binding molecule comprises:

an antibody VH region comprising: a VH CDR1 with an amino acid sequence selected from SEQ ID NO:1 and 7; a VH CDR2 with an amino acid sequence selected from SEQ ID NO:2 and 8; and a VH CDR3 with an amino acid sequence selected from SEQ ID NO:3 and 9; and an antibody VL region comprising: a VL CDR1 with an amino acid sequence selected from SEQ ID NO:4 and 10; a VL CDR2 with an amino acid sequence selected from SEQ ID NO:5 and 11; and a VL CDR3 with an amino acid sequence selected from SEQ ID NO:6 and 12; or

a variant thereof in which no more than one, two, three, four or five amino acid residues are altered by amino acid substitution, addition, or deletion, within the CDR sequences.

20. A binding molecule according to any of paragraphs 1 to 17, wherein the binding molecule comprises: a heavy chain variable region comprising: (a) a VH CDR1 region comprising SEQ ID NO: 1, or an amino acid sequence having one, two, three, four or five amino acid substitutions, deletions or additions as compared to SEQ ID NO: 1 ; (b) a VH CDR2 region comprising SEQ ID NO: 2, or an amino acid sequence having one, two, three, four or five amino acid substitutions, deletions or additions as compared to SEQ ID NO: 2; and (c) a VH CDR3 region comprising SEQ ID NO: 3, or an amino acid sequence having one, two, three, four or five amino acid substitutions, deletions or additions as compared to SEQ ID NO: 3; and/or a light chain variable region comprising: (a) a VL CDR1 region comprising SEQ ID NO: 4, or an amino acid sequence having one, two, three, four or five amino acid substitutions, deletions or additions as compared to SEQ ID NO: 4; (b) a VL CDR2 region comprising SEQ ID NO: 5, or an amino acid sequence having one, two, three, four or five amino acid substitutions, deletions or additions as compared to SEQ ID NO: 5; and (c) a VL CDR3 region comprising SEQ ID NO: 6, or an amino acid sequence having one, two, three, four or five amino acid substitutions, deletions or additions as compared to SEQ ID NO: 6.

21. A binding molecule according to any of paragraphs 1 to 17, wherein the binding molecule comprises: a heavy chain variable region comprising: (a) a VH CDR1 region comprising SEQ ID NO: 7, or an amino acid sequence having one, two, three, four or five amino acid substitutions, deletions or additions as compared to SEQ ID NO: 7; (b) a VH CDR2 region comprising SEQ ID NO: 8, or an amino acid sequence having one, two, three, four or five amino acid substitutions, deletions or additions as compared to SEQ ID NO: 8; and (c) a VH CDR3 region comprising SEQ ID NO: 9, or an amino acid sequence having one, two, three, four or five amino acid substitutions, deletions or additions as compared to SEQ ID NO: 9; and/or a light chain variable region comprising: (a) a VL CDR1 region comprising SEQ ID NO: 10, or an amino acid sequence having one, two, three, four or five amino acid substitutions, deletions or additions as compared to SEQ ID NO: 10; (b) a VL CDR2 region comprising SEQ ID NO: 11, or an amino acid sequence having one, two, three, four or five amino acid substitutions, deletions or additions as compared to SEQ ID NO: 11; and (c) a VL CDR3 region comprising SEQ ID NO: 12, or an amino acid sequence having one, two, three, four or five amino acid substitutions, deletions or additions as compared to SEQ ID NO: 12.

22. A binding molecule according to any preceding paragraph, which excludes an antibody, or antigen-binding fragment thereof, that comprises any of VH CDRs1-3 (SEQ ID NOs:1-3 or 7-9) and VL CDRs1-3 (SEQ ID NOs:4-6 or 10-12) of antibody IMP761.

23. A binding molecule according to any preceding paragraph, which excludes an antibody, or antigen-binding fragment thereof, that comprises one, two, or three complementarity determining regions (CDRs) of an antibody heavy chain variable (VH) region of IMP761 (VH CDRs1-3: SEQ ID NOs:1-3 or 7-9), and/or one, two, or three CDRs of an antibody light chain variable (VL) region of IMP761 (VL CDRs1-3: SEQ ID NOs:4-6 or 10-12).

24. A binding molecule according to any preceding paragraph, which excludes:

an antibody, or antigen-binding fragment thereof, that comprises: an antibody VH region comprising: a VH CDR1 with an amino acid sequence selected from SEQ ID NO:1 and 7; a VH CDR2 with an amino acid sequence selected from SEQ ID NO:2 and 8; and a VH CDR3 with an amino acid sequence selected from SEQ ID NO:3 and 9; and an antibody VL region comprising: a VL CDR1 with an amino acid sequence selected from SEQ ID NO:4 and 10; a VL CDR2 with an amino acid sequence selected from SEQ ID NO:5 and 11; and a VL CDR3 with an amino acid sequence selected from SEQ ID NO:6 and 12; or

a variant thereof in which no more than one, two, three, four or five amino acid residues are altered by amino acid substitution, addition, or deletion, within the CDR sequences.

25. A binding molecule according to any preceding paragraph, which excludes an antibody, or antigen-binding fragment thereof, that comprises: a heavy chain variable region comprising: (a) a VH CDR1 region comprising SEQ ID NO: 1, or an amino acid sequence having one, two, three, four or five amino acid substitutions, deletions or additions as compared to SEQ ID NO: 1 ; (b) a VH CDR2 region comprising SEQ ID NO: 2, or an amino acid sequence having one, two, three, four or five amino acid substitutions, deletions or additions as compared to SEQ ID NO: 2; and (c) a VH CDR3 region comprising SEQ ID NO: 3, or an amino acid sequence having one, two, three, four or five amino acid substitutions, deletions or additions as compared to SEQ ID NO: 3; and/or a light chain variable region comprising: (a) a VL CDR1 region comprising SEQ ID NO: 4, or an amino acid sequence having one, two, three, four or five amino acid substitutions, deletions or additions as compared to SEQ ID NO: 4; (b) a VL CDR2 region comprising SEQ ID NO: 5, or an amino acid sequence having one, two, three, four or five amino acid substitutions, deletions or additions as compared to SEQ ID NO: 5; and (c) a VL CDR3 region comprising SEQ ID NO: 6, or an amino acid sequence having one, two, three, four or five amino acid substitutions, deletions or additions as compared to SEQ ID NO: 6.

26. A binding molecule according to any preceding paragraph, which excludes an antibody, or antigen-binding fragment thereof, that comprises: a heavy chain variable region comprising: (a) a VH CDR1 region comprising SEQ ID NO: 7, or an amino acid sequence having one, two, three, four or five amino acid substitutions, deletions or additions as compared to SEQ ID NO: 7; (b) a VH CDR2 region comprising SEQ ID NO: 8, or an amino acid sequence having one, two, three, four or five amino acid substitutions, deletions or additions as compared to SEQ ID NO: 8; and (c) a VH CDR3 region comprising SEQ ID NO: 9, or an amino acid sequence having one, two, three, four or five amino acid substitutions, deletions or additions as compared to SEQ ID NO: 9; and/or a light chain variable region comprising: (a) a VL CDR1 region comprising SEQ ID NO: 10, or an amino acid sequence having one, two, three, four or five amino acid substitutions, deletions or additions as compared to SEQ ID NO: 10; (b) a VL CDR2 region comprising SEQ ID NO: 11, or an amino acid sequence having one, two, three, four or five amino acid substitutions, deletions or additions as compared to SEQ ID NO: 11; and (c) a VL CDR3 region comprising SEQ ID NO: 12, or an amino acid sequence having one, two, three, four or five amino acid substitutions, deletions or additions as compared to SEQ ID NO: 12.

27. A binding molecule according to any preceding paragraph, which excludes an antibody, or antigen-binding fragment thereof, that comprises VH CDRs1-3 and VL CDRs1-3 of antibody IMP761.

28. A pharmaceutical composition comprising a binding molecule according to any preceding paragraph, and a pharmaceutically acceptable carrier, excipient, or diluent.

29. A binding molecule according to any of paragraphs 1 to 27, or a pharmaceutical composition according to paragraph 28, for use as a medicament.

30. A binding molecule according to any of paragraphs 1 to 27, or a pharmaceutical composition according to paragraph 28, for use in the treatment of a T cell-mediated immune disorder.

31. Use of a binding molecule according to any of paragraphs 1 to 27, or a pharmaceutical composition according to paragraph 28, in the manufacture of a medicament for the treatment of a T cell-mediated immune disorder.

32. A method of treating a T cell-mediated immune disorder, which comprises administering an effective amount of a binding molecule according to any of paragraphs 1 to 27, or a pharmaceutical composition according to paragraph 28, to a subject in need of such treatment.

33. A binding molecule or a pharmaceutical composition for use according to paragraph 30, use according to paragraph 31, or a method according to paragraph 32, wherein the T cell-mediated immune disorder is treated, or to be treated, by inhibition of TCR-mediated signal transduction in LAG-3 positive T cells through agonism of LAG-3 by the binding molecule.

34. A binding molecule or a pharmaceutical composition for use according to paragraph 33, use according to paragraph 33, or a method according to paragraph 33, wherein the T cell-mediated immune disorder is treated, or to be treated, by inhibition of a calcineurin-NFAT signalling pathway in LAG-3 positive T cells through agonism of LAG-3 by the binding molecule.

35. A binding molecule or a pharmaceutical composition for use according to paragraph 33 or 34, use according to paragraph 33 or 34, or a method according to paragraph 33 or 34, wherein the T cell-mediated immune disorder is treated, or to be treated, by inhibiting expression of one or more NFAT-regulated genes through agonism of LAG-3 by the binding molecule.

36. A binding molecule or a pharmaceutical composition for use according to paragraph 35, use according to paragraph 35, or a method according to paragraph 35, wherein the one or more NFAT-regulated genes include genes encoding cytokines, receptors, or nuclear factors.

37. A binding molecule or a pharmaceutical composition for use according to paragraph 35 or 36, use according to paragraph 35 or 36, or a method according to paragraph 35 or 36, wherein the one or more NFAT-regulated genes include genes encoding IL-2, IFN-y, IL-4, IL-6, IL-17, IL-21, IL-22, IL-23, CD25, PD-1, or TIM-3.

38. A binding molecule or a pharmaceutical composition for use according to any of paragraphs 35 to 37, use according to any of paragraphs 35 to 37, or a method according to any of paragraphs 35 to 37, wherein the one or more NFAT-regulated genes includes a gene encoding IL-2.

39. A binding molecule or a pharmaceutical composition for use according to any of paragraphs 30 or 33 to 38, use according to any of paragraphs 31 or 33 to 38, or a method according to any of paragraphs 32 to 38, wherein the T-cell-mediated immune disorder is an inflammatory disease, or an autoimmune disorder.

40. A binding molecule or a pharmaceutical composition for use according to any of paragraphs 30 or 33 to 39, use according to any of paragraphs 31 or 33 to 39, or a method according to any of paragraphs 32 to 39, wherein the T-cell-mediated immune disorder is selected from the group consisting of infections (viral, bacterial, fungal and parasitic), endotoxic shock associated with infection, sepsis, arthritis, rheumatoid arthritis, asthma, COPD, pelvic inflammatory disease, Alzheimer's Disease, inflammatory bowel disease, Crohn's disease, ulcerative colitis, Peyronie's Disease, coeliac disease, gallbladder disease, Pilonidal disease, peritonitis, psoriasis, vasculitis, surgical adhesions, stroke, Type I Diabetes, lyme disease, arthritis, meningoencephalitis, autoimmune uveitis, immune mediated inflammatory disorders of the central and peripheral nervous system such as multiple sclerosis, lupus (such as systemic lupus erythematosus) and Guillain-Barré syndrome, Atopic dermatitis, autoimmune hepatitis, fibrosing alveolitis, Grave's disease, IgA nephropathy, idiopathic thrombocytopenic purpura, Meniere's disease, pemphigus, primary biliary cirrhosis, sarcoidosis, scleroderma, Wegener's granulomatosis, other autoimmune disorders, pancreatitis, trauma (surgery), graft-versus-host disease, transplant rejection, heart disease including ischaemic diseases such as myocardial infarction as well as atherosclerosis, intravascular coagulation, bone resorption, osteoporosis, osteoarthritis, periodontitis and hypochlorhydia, or infertility related to lack of fetal-maternal tolerance.

41. Use of an agonist of LAG-3 to inhibit TCR-mediated signal transduction in a LAG-3 positive T cell.

42. Use according to paragraph 41, to inhibit a calcineurin-NFAT signalling pathway in the LAG-3 positive T cell.

43. Use according to paragraph 41 or 42, to inhibit expression of a NFAT-regulated gene.

44. Use according to paragraph 43, wherein the NFAT-regulated gene is a gene encoding a cytokine, receptor, or nuclear factor.

45. Use according to paragraph 43 or 44, wherein the NFAT-regulated gene is a gene encoding IL-2.

46. Use according to any of paragraphs 41 to 45, wherein the agonist of LAG-3 is a binding molecule according to any of paragraphs 1 to 27.

47. Use according to any of paragraphs 41 to 46, wherein the agonist of LAG-3 is IMP761 or an antigen-binding fragment thereof.

48. Use according to any of paragraphs 41 to 47 which is an *in vitro* method.

Further aspects of the invention are defined in the numbered paragraphs below:

1. An isolated binding molecule, which is an agonist of LAG-3, and which inhibits TCR-mediated signal transduction in LAG-3 positive T cells through agonism of LAG-3.

2. A binding molecule according to paragraph 1, which inhibits a calcineurin-NFAT signalling pathway in LAG-3 positive T cells through agonism of LAG-3.

3. A binding molecule according to paragraph 1 or 2, which inhibits expression of one or more NFAT-regulated genes.

4. A binding molecule according to paragraph 3, wherein the one or more NFAT-regulated genes include genes encoding cytokines, receptors, or nuclear factors.

5. A binding molecule according to paragraph 3 or 4, wherein the one or more NFAT-regulated genes include genes encoding IL-2, IFN-y, IL-4, IL-6, IL-17, IL-21, IL-22, IL-23, CD25, PD-1, or TIM-3.

6. A binding molecule according to any of paragraphs 3 to 5, wherein the one or more NFAT-regulated genes includes a gene encoding IL-2.

7. A binding molecule according to any of paragraphs 1 to 6, which inhibits antigen-induced CD4$^+$ and/or CD8$^+$ T-cell proliferation.

8. A binding molecule according to any of paragraphs 1 to 7, which inhibits antigen-induced CD4$^+$ and/or CD8$^+$ T-cell activation.

9. A binding molecule according to any of paragraphs 1 to 8, which binds to a human LAG-3 protein (or a human LAG-3Ig protein) with a dissociation constant ($K_D$) of no more than 100 pM, no more than 90 pM, no more than 80 pM, no more than 70 pM, no more than 60 pM, no more than 50 pM, no more than 40 pM, no more than 30 pM, or no more than 25 pM, for example as determined by Biacore analysis.

10. A binding molecule according to any of paragraphs 1 to 9, wherein the binding molecule comprises an antibody, or an antigen-binding fragment thereof.

11. A binding molecule according to any of paragraphs 1 to 10, wherein the binding molecule comprises one, two, or three complementarity determining regions (CDRs) of an antibody heavy chain variable (VH) region of IMP761 (VH CDRs1-3: SEQ ID NOs:1-3 or 7-9), and/or one, two, or three CDRs of an antibody light chain variable (VL) region of IMP761 (VL CDRs1-3: SEQ ID NOs:4-6 or 10-12).

12. A binding molecule according to any of paragraphs 1 to 10, wherein the binding molecule comprises:

an antibody VH region comprising:

a VH CDR1 with an amino acid sequence selected from SEQ ID NO:1 and 7;

a VH CDR2 with an amino acid sequence selected from SEQ ID NO:2 and 8; and

a VH CDR3 with an amino acid sequence selected from SEQ ID NO:3 and 9; and

an antibody VL region comprising:

a VL CDR1 with an amino acid sequence selected from SEQ ID NO:4 and 10;

a VL CDR2 with an amino acid sequence selected from SEQ ID NO:5 and 11; and

a VL CDR3 with an amino acid sequence selected from SEQ ID NO:6 and 12; or

a variant thereof in which no more than one, two, three, four or five amino acid residues are altered by amino acid substitution, addition, or deletion, within the CDR sequences.

13. A binding molecule according to any of paragraphs 1 to 10, wherein the binding molecule comprises:

a heavy chain variable region comprising:

(a) a VH CDR1 region comprising SEQ ID NO: 1, or an amino acid sequence having one, two, three, four or five amino acid substitutions, deletions or additions as compared to SEQ ID NO: 1;

(b) a VH CDR2 region comprising SEQ ID NO: 2, or an amino acid sequence having one, two, three, four or five amino acid substitutions, deletions or additions as compared to SEQ ID NO: 2; and

(c) a VH CDR3 region comprising SEQ ID NO: 3, or an amino acid sequence having one, two, three, four or five amino acid substitutions, deletions or additions as compared to SEQ ID NO: 3; and/or

a light chain variable region comprising:

(a) a VL CDR1 region comprising SEQ ID NO: 4, or an amino acid sequence having one, two, three, four or five amino acid substitutions, deletions or additions as compared to SEQ ID NO: 4;

(b) a VL CDR2 region comprising SEQ ID NO: 5, or an amino acid sequence having one, two, three, four or five amino acid substitutions, deletions or additions as compared to SEQ ID NO: 5; and

(c) a VL CDR3 region comprising SEQ ID NO: 6, or an amino acid sequence having one, two, three, four or five amino acid substitutions, deletions or additions as compared to SEQ ID NO: 6.

14. A binding molecule according to any of paragraphs 1 to 10, wherein the binding molecule comprises:

a heavy chain variable region comprising:

(a) a VH CDR1 region comprising SEQ ID NO: 7, or an amino acid sequence having one, two, three, four or five amino acid substitutions, deletions or additions as compared to SEQ ID NO: 7;

(b) a VH CDR2 region comprising SEQ ID NO: 8, or an amino acid sequence having one, two, three, four or five amino acid substitutions, deletions or additions as compared to SEQ ID NO: 8; and

(c) a VH CDR3 region comprising SEQ ID NO: 9, or an amino acid sequence having one, two, three, four or five amino acid substitutions, deletions or additions as compared to SEQ ID NO: 9; and/or

a light chain variable region comprising:

(a) a VL CDR1 region comprising SEQ ID NO: 10, or an amino acid sequence having one, two, three, four or five amino acid substitutions, deletions or additions as compared to SEQ ID NO: 10;

(b) a VL CDR2 region comprising SEQ ID NO: 11, or an amino acid sequence having one, two, three, four or five amino acid substitutions, deletions or additions as compared to SEQ ID NO: 11; and

(c) a VL CDR3 region comprising SEQ ID NO: 12, or an amino acid sequence having one, two, three, four or five amino acid substitutions, deletions or additions as compared to SEQ ID NO: 12.

15. A binding molecule according to any of paragraphs 1 to 10, which excludes an antibody, or antigen-binding fragment thereof, that comprises any of VH CDRs1-3 (SEQ ID NOs:1-3 or 7-9) and VL CDRs1-3 (SEQ ID NOs:4-6 or 10-12) of antibody IMP761.

16. A binding molecule according to any of paragraphs 1 to 10, which excludes an antibody, or antigen-binding fragment thereof, that comprises one, two, or three complementarity determining regions (CDRs) of an antibody heavy chain variable (VH) region of IMP761 (VH CDRs1-3: SEQ ID NOs:1-3 or 7-9), and/or one, two, or three CDRs of an antibody light chain variable (VL) region of IMP761 (VL CDRs1-3: SEQ ID NOs:4-6 or 10-12).

17. A binding molecule according to any of paragraphs 1 to 10, which excludes an antibody, or antigen-binding fragment thereof, that comprises:

an antibody VH region comprising:

a VH CDR1 with an amino acid sequence selected from SEQ ID NO:1 and 7;

a VH CDR2 with an amino acid sequence selected from SEQ ID NO:2 and 8; and

a VH CDR3 with an amino acid sequence selected from SEQ ID NO:3 and 9; and

an antibody VL region comprising:

a VL CDR1 with an amino acid sequence selected from SEQ ID NO:4 and 10;

a VL CDR2 with an amino acid sequence selected from SEQ ID NO:5 and 11; and

a VL CDR3 with an amino acid sequence selected from SEQ ID NO:6 and 12; or

a variant thereof in which no more than one, two, three, four or five amino acid residues are altered by amino acid substitution, addition, or deletion, within the CDR sequences.

18. A binding molecule according to any of paragraphs 1 to 10, which excludes an antibody, or antigen-binding fragment thereof, that comprises:

a heavy chain variable region comprising:

(a) a VH CDR1 region comprising SEQ ID NO: 1, or an amino acid sequence having one, two, three, four or five amino acid substitutions, deletions or additions as compared to SEQ ID NO: 1;

(b) a VH CDR2 region comprising SEQ ID NO: 2, or an amino acid sequence having one, two, three, four or five amino acid substitutions, deletions or additions as compared to SEQ ID NO: 2; and

(c) a VH CDR3 region comprising SEQ ID NO: 3, or an amino acid sequence having one, two, three, four or five amino acid substitutions, deletions or additions as compared to SEQ ID NO: 3; and/or

a light chain variable region comprising:

(a) a VL CDR1 region comprising SEQ ID NO: 4, or an amino acid sequence having one, two, three, four or five amino acid substitutions, deletions or additions as compared to SEQ ID NO: 4;

(b) a VL CDR2 region comprising SEQ ID NO: 5, or an amino acid sequence having one, two, three, four or five amino acid substitutions, deletions or additions as compared to SEQ ID NO: 5; and

(c) a VL CDR3 region comprising SEQ ID NO: 6, or an amino acid sequence having one, two, three, four or five amino acid substitutions, deletions or additions as compared to SEQ ID NO: 6.

19. A binding molecule according to any of paragraphs 1 to 10, which excludes an antibody, or antigen-binding fragment thereof, that comprises:

a heavy chain variable region comprising:

(a) a VH CDR1 region comprising SEQ ID NO: 7, or an amino acid sequence having one, two, three, four or five amino acid substitutions, deletions or additions as compared to SEQ ID NO: 7;

(b) a VH CDR2 region comprising SEQ ID NO: 8, or an amino acid sequence having one, two, three, four or five amino acid substitutions, deletions or additions as compared to SEQ ID NO: 8; and

(c) a VH CDR3 region comprising SEQ ID NO: 9, or an amino acid sequence having one, two, three, four or five amino acid substitutions, deletions or additions as compared to SEQ ID NO: 9; and/or

a light chain variable region comprising:

(a) a VL CDR1 region comprising SEQ ID NO: 10, or an amino acid sequence having one, two, three, four or five amino acid substitutions, deletions or additions as compared to SEQ ID NO: 10;

(b) a VL CDR2 region comprising SEQ ID NO: 11, or an amino acid sequence having one, two, three, four or five amino acid substitutions, deletions or additions as compared to SEQ ID NO: 11; and

(c) a VL CDR3 region comprising SEQ ID NO: 12, or an amino acid sequence having one, two, three, four or five amino acid substitutions, deletions or additions as compared to SEQ ID NO: 12.

20. A binding molecule according to any of paragraphs 1 to 11, which excludes an antibody, or antigen-binding fragment thereof, that comprises VH CDRs1-3 and VL CDRs1-3 of antibody IMP761.

21. A pharmaceutical composition comprising a binding molecule according to any of paragraphs 1 to 20, and a pharmaceutically acceptable carrier, excipient, or diluent.

22. A binding molecule according to any of paragraphs 1 to 20, or a pharmaceutical composition according to paragraph 21, for use as a medicament.

23. A binding molecule according to any of paragraphs 1 to 20, or a pharmaceutical composition according to paragraph 21, for use in the treatment of a T cell-mediated immune disorder.

24. Use of a binding molecule according to any of paragraphs 1 to 20, or a pharmaceutical composition according to paragraph 21, in the manufacture of a medicament for the treatment of a T cell-mediated immune disorder.

25. A method of treating a T cell-mediated immune disorder, which comprises administering an effective amount of a binding molecule according to any of paragraphs 1 to 20, or a pharmaceutical composition according to paragraph 21, to a subject in need of such treatment.

26. A binding molecule or a pharmaceutical composition for use according to paragraph 23, use according to paragraph 24, or a method according to paragraph 25, wherein the T cell-mediated immune disorder is treated, or to be treated, by inhibition of TCR-mediated signal transduction in LAG-3 positive T cells through agonism of LAG-3 by the binding molecule.

27. A binding molecule or a pharmaceutical composition for use according to paragraph 26, use according to paragraph 26, or a method according to paragraph 26, wherein the T cell-mediated immune disorder is treated, or to be treated, by inhibition of a calcineurin-NFAT signalling pathway in LAG-3 positive T cells through agonism of LAG-3 by the binding molecule.

28. A binding molecule or a pharmaceutical composition for use according to paragraph 26 or 27, use according to paragraph 26 or 27, or a method according to paragraph 26 or 27, wherein the T cell-mediated immune disorder is treated, or to be treated, by inhibiting expression of one or more NFAT-regulated genes through agonism of LAG-3 by the binding molecule.

29. A binding molecule or a pharmaceutical composition for use according to paragraph 28, use according to paragraph 28, or a method according to paragraph 28, wherein the one or more NFAT-regulated genes include genes encoding cytokines, receptors, or nuclear factors.

30. A binding molecule or a pharmaceutical composition for use according to paragraph 28 or 29, use according to paragraph 28 or 29, or a method according to paragraph 28 or 29, wherein the one or more NFAT-regulated genes include genes encoding IL-2, IFN-y, IL-4, IL-6, IL-17, IL-21, IL-22, IL-23, CD25, PD-1, or TIM-3.

31. A binding molecule or a pharmaceutical composition for use according to any of paragraphs 28 to 30, use according to any of paragraphs 28 to 30, or a method according to any of paragraphs 28 to 30, wherein the one or more NFAT-regulated genes includes a gene encoding IL-2.

32. A binding molecule or a pharmaceutical composition for use according to any of paragraphs 23 or 26 to 31, use according to any of paragraphs 24 or 26 to 31, or a method according to any of paragraphs 25 to 31, wherein the T-cell-mediated immune disorder is an inflammatory disease, or an autoimmune disorder.

33. A binding molecule or a pharmaceutical composition for use according to any of paragraphs 23 or 26 to 32, use according to any of paragraphs 24 or 26 to 32, or a method according to any of paragraphs 25 to 32, wherein the T-cell-mediated immune disorder is selected from the group consisting of infections (viral, bacterial, fungal and parasitic), endotoxic shock associated with infection, sepsis, arthritis, rheumatoid arthritis, asthma, COPD, pelvic inflammatory disease, Alzheimer's Disease, inflammatory bowel disease, Crohn's disease, ulcerative colitis, Peyronie's Disease, coeliac disease, gallbladder disease, Pilonidal disease, peritonitis, psoriasis, vasculitis, surgical adhesions, stroke, Type I Diabetes, lyme disease, arthritis, meningoencephalitis, autoimmune uveitis, immune mediated inflammatory disorders of the central and peripheral nervous system such as multiple sclerosis, lupus (such as systemic lupus erythematosus) and Guillain-Barré syndrome, Atopic dermatitis, autoimmune hepatitis, fibrosing alveolitis, Grave's disease, IgA nephropathy, idiopathic thrombocytopenic purpura, Meniere's disease, pemphigus, primary biliary cirrhosis, sarcoidosis, scleroderma, Wegener's granulomatosis, other autoimmune disorders, pancreatitis, trauma (surgery), graft-versus-host disease, transplant rejection, heart disease including ischaemic diseases such as myocardial infarction as well as atherosclerosis, intravascular coagulation, bone resorption, osteoporosis, osteoarthritis, periodontitis and hypochlorhydia, or infertility related to lack of fetal-maternal tolerance.

34. Use of an agonist of LAG-3 to inhibit TCR-mediated signal transduction in a LAG-3 positive T cell.

35. Use according to paragraph 34, to inhibit a calcineurin-NFAT signalling pathway in the LAG-3 positive T cell.

36. Use according to paragraph 34 or 35, to inhibit expression of a NFAT-regulated gene.

37. Use according to paragraph 36, wherein the NFAT-regulated gene is a gene encoding a cytokine, receptor, or nuclear factor.

38. Use according to paragraph 36 or 37, wherein the NFAT-regulated gene is a gene encoding IL-2.

39. Use according to any of paragraphs 34 to 38, wherein the agonist of LAG-3 is a binding molecule according to any of paragraphs 1 to 20.

40. Use according to any of paragraphs 34 to 39, wherein the agonist of LAG-3 is IMP761 or an antigen-binding fragment thereof.

Embodiments of the invention are now described, by way of example only, with reference to the accompanying drawings in which:

Figure 1 shows a schematic overview of TCR signalling (from Belikov, Aleksey. (2016). *The role of reactive oxygen species and mitochondria in T-cell activation.* 10.13140/RG.2.1.2916.0568);

Figure 2 illustrates receptors (CD4, TCR, LAG-3) on T cells which are known to bind MHC class II molecules, and shows known signalling pathways for CD4 and the TCR, but the LAG-3 signalling pathway is unknown;

Figure 3 shows amino acid sequence (SEQ ID NO:13) of mature human LAG-3 protein. The four extracellular Ig superfamily domains are at amino acid residues: 1-149 (D1); 150-239 (D2); 240-330 (D3); and 331-412 (D4). The amino acid sequence of the extra-loop structure of the D1 domain of human LAG-3 protein is shown underlined in bold (SEQ ID NO:18). The amino acid residues of the discontinuous epitope of LAG-3 bound by IMP761 antibody are highlighted with grey shading;

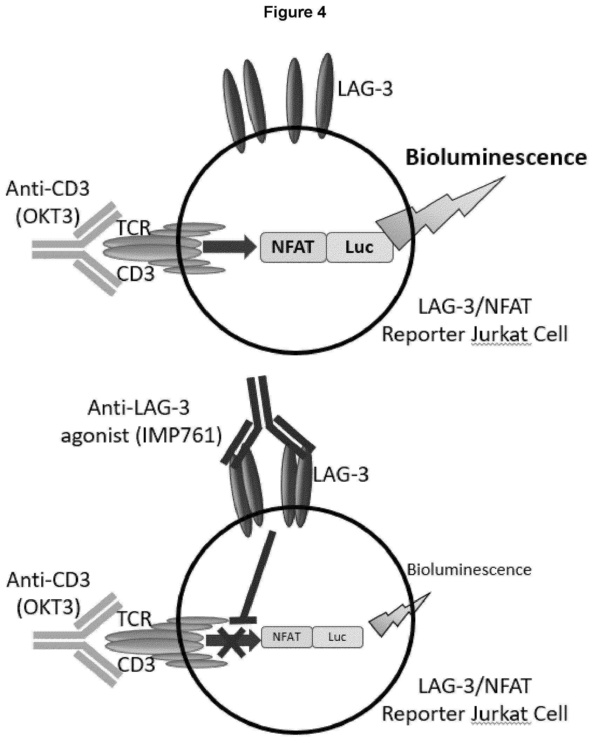

Figure 4 shows schematically the mode of action of a LAG-3 agonist (IMP761) in the inhibition of expression of a luciferase reporter gene under the control of a NFAT response element in the presence of anti-CD3 antibody (OKT3). Anti-CD3 antibody binds to CD3 on the surface of a Reporter Jurkat Cell, leading to activation of expression of luciferase from the NFAT/Luc reporter gene through TCR-mediated signal transduction (upper part). Binding of IMP761 to LAG-3 on the surface of the Reporter Jurkat Cell inhibits TCR-mediated signal transduction, leading to downregulation of expression of luciferase from the NFAT/Luc reporter gene (lower part);

Figure 5 shows the results of an assay demonstrating that a LAG-3 agonist (IMP761) inhibits expression in a LAG-3 positive Jurkat T cell of a luciferase reporter gene under the control of a NFAT response element, compared with a negative control antibody (IgG4);

Figure 6 shows ramp rendering of a 3D homology model of the Ig-like V domain (aa_37-167) of human LAG3;

Figure 7 shows (a) the CLIPS reaction; and (b) CLIPS constructs with a range of structures produced from native sequences. From left to right: two different single T2 loops, T3 double loop, conjugated T2+T3 loops, stabilized beta sheet, and stabilized alpha helix;

Figure 8 shows the target protein (left) containing a discontinuous conformational epitope, which is converted into a matrix library (middle). Combinatorial peptides are synthesized on a minicard and chemically converted into spatially defined CLIPS constructs (right);

Figure 9 shows T3 looped CLIPS™ construct;

Figure 10 shows heat map methodology: A. Table of combined peptides, with two sub-sequences indicated as "Loop 1" and "Loop 2". B. Data from A displayed as a matrix. C. Greyscale bar indication of the heat map representation. D. Heat map visualization of data from A;

Figure 11. Box plot graphs of raw data of antibody screening. The bottom and top of the boxes are the 25th and 75th percentile of the data. The band near the middle of the box is the 50th percentile (the median). The whiskers are at 1.5 the inter-quantile range, an indication of statistical outliers within the dataset (*ref Mcgill et al., The American Statistician,* 32: 12-16, 1978);

Figure 12 shows a heatmap representation of intensity profile recorded for monoclonal antibody IMP761 on set 6. Signals of high intensity are plotted in grey and background signal is plotted in black; and

Figure 13 shows 3D visualizations of epitope candidates identified in this study obtained using a homology model shown in Figure 6. Cartoon representation is shown in the left panel and surface representation in the right panel (same orientation). Discontinuous epitope identified for IMP761 is displayed. Peptide stretches $_{47}$TIPLQDLSLLRR$_{58}$, $_{107}$GGLRSGRLPLQ$_{117}$, $_{128}$QRGDFSLWLRPAR$_{140}$, $_{153}$LRDRALSCRL$_{162}$ are each shaded differently.

## Example 1 - LAG-3 Agonist Antibody Inhibits NFAT-Regulated Gene Expression

This example demonstrates that an agonist of LAG-3 (IMP761 antibody) inhibits expression of a reporter gene (luciferase) under the control of a NFAT response element in a LAG-3 positive T cell.

The method is based on the capacity of IMP761 to decrease the activation of a LAG-3 effector cell line induced by a low dose of an anti-CD3 antibody (OKT3 clone, 3ng/ml), mimicking antigen-stimulation of T cells. The LAG-3 effector cell line is a Jurkat T cell line expressing LAG-3 at the surface, and containing the luciferase gene under the control of an NFAT response element *(Jurkat Lag-3$^+$/NFAT-luc2 effector cells,* from Promega). After binding to LAG-3 expressed on the surface of the Jurkat cells, IMP761 triggers downregulation of TCR-induced NFAT-regulated expression (illustrated schematically in Figure 4, lower part). Luciferase activity of the cell line is used to measure TCR-driven cell activation which is dampened in the presence of IMP761.

Reagents

Jurkat LAG-3+/NFAT-luc2 effector cells (Promega, ref: CS194801)
RPMI 1640 (GIBCO, ref: 31870-025)
L-Glutamine (200 mM) (GIBCO, ref: 25030-024)
HEPES (1 M) (GIBCO, ref: 15630-080)
FCS (GIBCO, ref: 10270106)
IMP761 (2.06 mg/ml) (IMMUTEP, lot: 270416)
human IgG4, control (Biolegend, ref: 403402)
Anti-CD3 (OKT3) (eBioscience, ref: 16-0037-85)
Bio-Glo reagent (PROMEGA, ref: G7941)
White, 96-well solid flat-bottom microplates (COSTAR, ref: 3917)
Assay medium: RPMI 1640, L-Glutamine (2mM), Hepes (10 mM), FCS 1%
Cell concentration: $1.33 \times 10^6$ cells/ml

Protocol

1. Pass Jurkat LAG-3+/NFAT-luc2 effector cells at day -1 before assay in order to have a cell density around 1Million/ml (between 0.8 and 1.2 Million/ml) at day 0.
2. Prepare assay medium if needed and pre-warm medium for 30 minutes at 37°C:

| FCS | 0.5 ml |
| --- | --- |
| L-Glutamine (200mM) | 0.5 ml |
| HEPES (1M) | 0.5 ml |
| RPMI 1640 | 48.5 ml |

3. Prepare IMP761 quality control (QC) stock solution if needed:

Make 24,000 ng/ml stock solution; for example:
10ml of stock IMP761 (2.06 mg/ml) + 848.3 μl Assay medium.

Store 25 μl aliquots in -80$^0$C freezer.

4. Prepare 3X solutions of IMP761 (batch 270416 at 2.06 mg/ml), human IgG4 negative control (or any other antibody). Adapt the predilution steps according to the initial concentration of the antibody: the volume of assay medium (in μl) to be added to dilute 2 μl of a stock solution in order to prepare a 100 μg/ml predilution is:

$$\left(2 \; x \left( \frac{Initial\; concentration\; (in\; mg/ml)}{0.1} \right) - 2 \right) \; \mu l \text{ of assay medium}$$

| IMP761 Dilution to prepare 3X working solutions | | | |
|---|---|---|---|
| Predilution (100 µg/ml) | 2 µl of stock (2.06 mg/ml) + 39.2 µl of assay medium | | Final concentration (ng/ml) |
| 2000 ng/ml | 10 µl of 100 µg/ml predilution + 490 µl of assay medium | | 666.7 |
| 1000ng/ml | 200 µl of 2000 ng/ml dilution + 200 µl of assay medium | | 333.3 |
| 500 ng/ml | 200 µl of 1000 ng/ml dilution + 200 µl of assay medium | | 166.7 |
| 250 ng/ml | 200 µl of 500 ng/ml dilution + 200 µl of assay medium | | 83.3 |
| 125 ng/ml | 200 µl of 250 ng/ml dilution + 200 µl of assay medium | | 41.7 |
| 62.5 ng/ml | 200 µl of 125 ng/ml dilution + 200 µl of assay medium | | 20.8 |
| 31.2ng/ml | 200 µl of 62.5 ng/ml dilution + 200 µl of assay medium | | 10.4 |
| 15.6ng/ml | 200 µl of 31.2 ng/ml dilution + 200 µl of assay medium | | 5.2 |
| 7.8 ng/ml | 200 µl of 15.6 ng/ml dilution + 200 µl of assay medium | | 2.6 |

(Serial Dilutions)

5. Prepare 3X IMP761 quality control (QC) solutions
Thaw stock IMP761 QC [24,000 ng/ml] aliquot and make serial dilutions

Very High (VH) QC:     20 µl      [24,000 ng/ml] + 380 µl = 1,200 ng/ml (final concentration: 400 ng/ml)
High (H) QC:           150 µl     [1,200 ng/ml] + 225 µl = 480 ng/ml (final concentration: 160 ng/ml)
Medium (M) QC:         150 µl     [480 ng/ml] + 225 µl = 192 ng/ml (final concentration: 64 ng/ml)
Low (L) QC:            150 µl     [192 ng/ml] + 225 µl = 76.8 ng/ml (final concentration: 25.6 ng/ml)
Very Low (VL) QC:      150 µl     [25.6 ng/ml] + 225 µl = 10.24 ng/ml (final concentration: 3.4 ng/ml)

6. Prepare OKT3 reagent

3.6 µl stock + 4 ml assay medium = 0.9 µg/ml

150 µl of [0.9 µg/ml] + 14,850 µl of assay medium = 9 ng/ml (3X)

7. Prepare Jurkat LAG-3/NFAT-luc2 effector cells

At day 0, count cells using Trypan Blue staining
Centrifuge cells at 1200 rpm for 5 minutes
Aspirate media and resuspend cells at $3.75 \times 10^6$/ml in assay medium.

8. Distribute 3X solutions in 96 well plate
Do not use outer wells because of edge effect
Distribute 40 µl of 3X solutions of IMP761 / QC / assay medium (0) into the corresponding wells of the assay plate in duplicate
Distribute 40 µl/well of 3X solution of anti-CD3 / assay medium (Unstimulated control: unstim) into each well of the assay plate in duplicate. Final concentration: 3 ng/ml
Distribute 40 µl per well ($0.15 \times 10^6$/well)
Example of plate template (Reference batch/unknown)

|  |  |  |  |  |  |  |  |  |  |  |  |
|---|---|---|---|---|---|---|---|---|---|---|---|
|  | 666.7 | 333.3 | 166.7 | 83.3 | 41.7 | 20.8 | 10.4 | 5.2 | 2.6 | 0 |  |
|  | 666.7 | 333.3 | 166.7 | 83.3 | 41.7 | 20.8 | 10.4 | 5.2 | 2.6 | 0 |  |
|  | 666.7 | 333.3 | 166.7 | 83.3 | 41.7 | 20.8 | 10.4 | 5.2 | 2.6 | 0 |  |
|  | 666.7 | 333.3 | 166.7 | 83.3 | 41.7 | 20.8 | 10.4 | 5.2 | 2.6 | 0 |  |
|  | QC VHIGH | QC HIGH | QC MID | QC LOW | QC VLOW | UNSTIM |  |  |  |  |  |

(continued)

| | QC VHIGH | QC HIGH | QC MID | QC LOW | QC VLOW | UNSTIM | | | | | |
|---|---|---|---|---|---|---|---|---|---|---|---|
| | | | | | | | | | | | |

9. Incubate plate(s) at 37°C for 24 hours

10. Prepare BioGlo reagent

a) Place frozen BioGlo reagent at room temperature (RT) 3-6 hours before use to allow thawing

b) Transfer the buffer (10 ml) to substrate bottle, mix and keep at RT in the dark until use

11. Equilibrate plate(s) at RT for 15 minutes

12. Add 120 μl / well of BioGlo, avoid / remove bubbles

13. Incubate at RT for 5 to 15 minutes

14. Measure luminescence (RLU), integration time = 0.5 sec/well using PerkinElmer 2103 Multilabel reader Envision. 3 measures for each well are collected 45 seconds apart, calculate the average of 3 measures to obtain "RLU Average".

Results

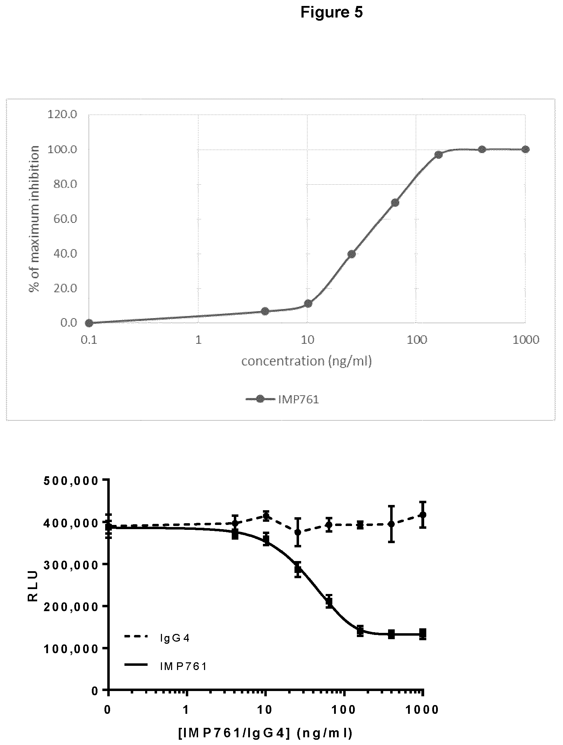

Figure 5 shows the results obtained, using IgG4 antibody as a negative control. Maximum activation was recorded as the activation observed when no IMP761 was present. Maximum inhibition was recorded as the activation observed when IMP761 concentration was 1000 ng/ml. A five-parameter non-linear regression model determined that the $IC_{50}$ of the IMP761 was 37 ng/ml.

## Example 2 - Full discontinuous epitope mapping of anti-LAG-3 antibody IMP761

This example describes mapping of the epitope of antibody IMP761 targeting human LAG-3 by means of full discontinuous epitope mapping.

Pepscan analysis established an epitope candidate for antibody IMP761. The antibody recognizes a complex discontinuous epitope within the Ig-like V domain of human LAG3.

## Materials

Antibody

IMP761: recognises human LAG-3. Human Fc 0.52 mg/ml.

Target Protein

> sp|P18627|LAG3_HUMAN Lymphocyte activation gene 3 protein OS=Homo sapiens GN=LAG3 PE=1 SV=5

```
  1                                         VP VVWAQEGAPA QLPCSPTIPL  50
 51 QDLSLLRRAG VTWQHQPDSG PPAAAPGHPL APGPHPAAPS SWGPRPRRYT 100
101 VLSVGPGGLR SGRLPLQPRV QLDERGRQRG DFSLWLRPAR RADAGEYRAA 150
151 VHLRDRALSC RLRLRLGQAS MTASPPGSLR ASDWVILNCS FSRPDRPASV 200
201 HWFRNRGQGR VPVRESPHHH LAESFLFLPQ VSPMDSGPWG CILTYRDGFN 250
251 VSIMYNLTVL GLEPPTPLTV YAGAGSRVGL PCRLPAGVGT RSFLTAKWTP 300
301 PGGGPDLLVT GDNGDFTLRL EDVSQAQAGT YTCHIHLQEQ QLNATVTLAI 350
351 ITVTPKSFGS PGSLGKLLCE VTPVSGQERF VWSSLDTPSQ RSFSGPWLEA 400
401 QEAQLLSQPW QCQLYQGERL LGAAVYFTEL SSPGAQRSGR APGALPAGHL 450
451                                                          500
501                             525 (SEQ ID NO:24)
```

*) Residues 1-28 and 451 and further, stricken though in the sequence above correspond to presequence or TM helix and intracellular domain of LAG-3, and were not used for array synthesis.

Note that the first 22 amino acid residues of the human LAG-3 sequence shown above (MWEAQFLGLLFLQPLWVAPVKP; SEQ ID NO:23) are not included in the human LAG-3 sequence shown in Figure 3, so the numbering of LAG-3 amino acid residues in the example below differs from the numbering of the sequence in Figure 3.

Data Visualization

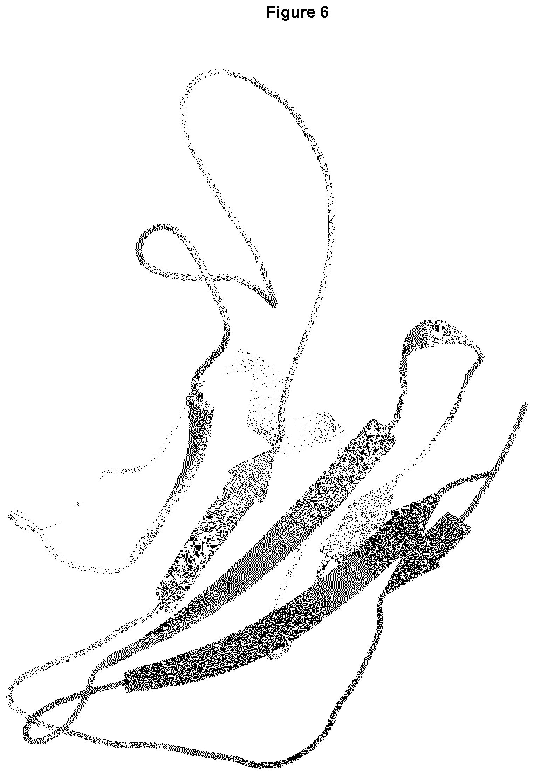

A homology model was built for the Ig-like V domain (aa_37-167) of human LAG-3 using 2ptt.pdb as a template (sequence identity about 20 % and secondary structure identity 56%). A ramp rendering of the 3D homology model is shown in Figure 6.

**Methods**

The Principles of Clips Technology

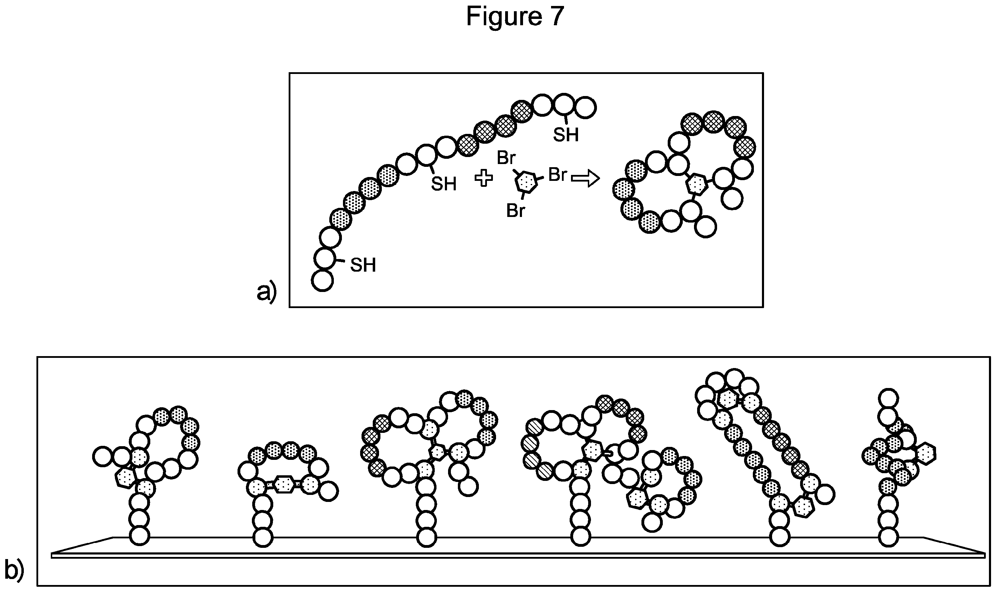

CLIPS technology structurally fixes peptides into defined three-dimensional structures. This results in functional mimics of even the most complex binding sites. CLIPS technology is used to shape peptide libraries into single, double or triple looped structures as well as sheet- and helix-like folds (see Figure 7).
The CLIPS reaction takes place between bromo groups of the CLIPS scaffold and thiol sidechains of cysteines. The reaction is fast and specific under mild conditions. Using this elegant chemistry, native protein sequences are transformed into CLIPS constructs with a range of structures. From left to right in Figure 7(b): two different single T2 loops, T3 double loop, conjugated T2+T3 loops, stabilized beta sheet, and stabilized alpha helix (Timmerman et al., J. Mol. Recognit. 2007; 20: 283-29).

Combinatorial CLIPS Library Screening in Detail

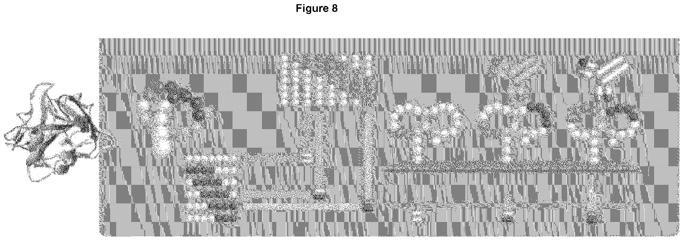

CLIPS library screening starts with the conversion of the target protein into a library of up to 10,000 overlapping peptide constructs, using a combinatorial matrix design. On a solid carrier, a matrix of linear peptides is synthesized, which are subsequently shaped into spatially defined CLIPS constructs (see Figure 8). Constructs representing both parts of the discontinuous epitope in the correct conformation bind the antibody with high affinity, which is detected and quantified. Constructs presenting the incomplete epitope bind the antibody with lower affinity, whereas constructs not containing the epitope do not bind at all. Affinity information is used in iterative screens to define the sequence and conformation of epitopes in detail.
The target protein (Figure 8, left) containing a discontinuous conformational epitope is converted into a matrix library (Figure 8, middle). Combinatorial peptides are synthesized on a minicard and chemically converted into spatially defined CLIPS constructs (Figure 8, right).

Heat Map Analysis

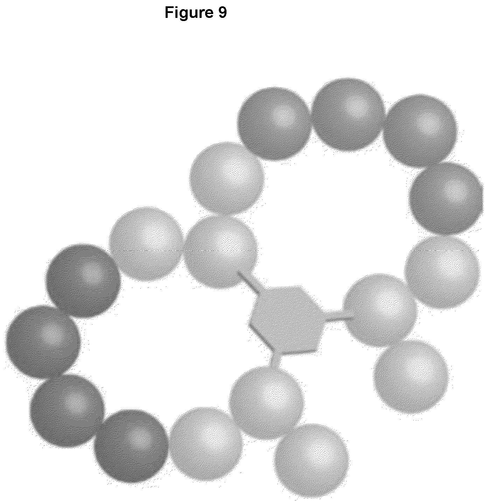

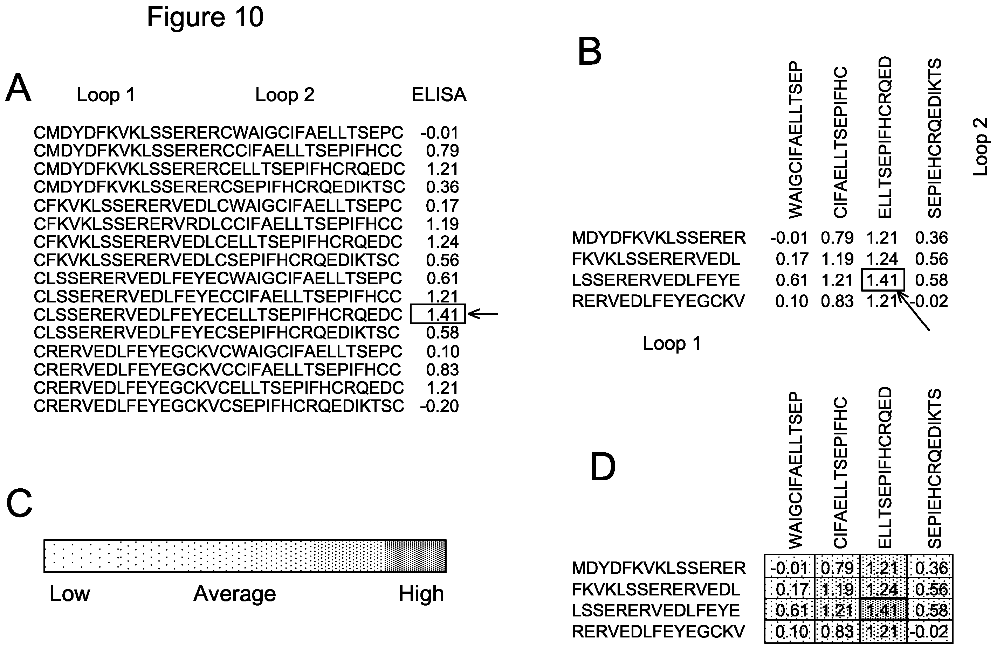

A heat map is a graphical representation of data where the values taken by a variable in a two-dimensional map are represented in different shades.
For double-looped CLIPS peptides, such a two-dimensional map can be derived from the independent sequences of the first and second loops. For example, the sequences of the 16 CLIPS peptides depicted in Figure 10 are effectively permutations of 4 unique sub-sequences in loop 1 (darker circles on the left in Figure 9) and 4 unique sub-sequences in loop 2 (darker circles on the right in Figure 9). Thus, the observed ELISA data (Figure 10A) can be plotted in a 4x4 matrix, where each X coordinate corresponds to the sequence of the first loop, and each Y coordinate corresponds to the sequence of the second loop. For instance, the ELISA value observed for CLIPS peptide CLSSERERVEDLFEYECELLTSEPIFHCRQEDC (SEQ ID NO:85; indicated with an arrow in Figure 10A) can be found at the third row, third column of Figure 10B (indicated with an arrow and a square).
To further facilitate the visualization, ELISA values can be replaced with shades from a continuous gradient. In this case, extremely low values are lightly shaded, extremely high values are more darkly shaded, and average values are in black (see Figure 10C). For the aforementioned example, the average value is 0.71. When this shading is applied to the data matrix depicted in Figure 10B, a shaded heat map is obtained (see Figure 10D, the original data is still indicated for extra clarity).

Synthesis of Peptides

To reconstruct epitopes of the target molecule a library of peptides was synthesized. An amino functionalized polypropylene support was obtained by grafting with a proprietary hydrophilic polymer formulation, followed by reaction with t-butyloxycarbonyl-hexamethylenediamine (BocHMDA) using dicyclohexylcarbodiimide (DCC) with Nhydroxybenzotriazole (HOBt) and subsequent cleavage of the Boc-groups using trifluoroacetic acid (TFA). Standard Fmoc-peptide synthesis was used to synthesize peptides on the amino-functionalized solid support by custom modified JANUS liquid handling stations (Perkin Elmer).

Synthesis of structural mimics was done using Chemically Linked Peptides on Scaffolds (CLIPS) technology. CLIPS technology allows to structure peptides into single loops, double loops, triple loops, sheet-like folds, helix-like folds and combinations thereof. CLIPS templates are coupled to cysteine residues. The side-chains of multiple cysteines in the peptides are coupled to one or two CLIPS templates. For example, a 0.5 mM solution of the P2 CLIPS (2,6-bis(bromomethyl)pyridine) is dissolved in ammonium bicarbonate (20 mM, pH 7.8)/acetonitrile (1:3(v/v)). This solution is added onto the peptide arrays. The CLIPS template will bind to side-chains of two cysteines as present in the solid-phase bound peptides of the peptide-arrays (455-well plate with 3 $\mu$l wells). The peptide arrays are gently shaken in the solution for 30 to 60 minutes while completely covered in solution. Finally, the peptide arrays are washed extensively with excess of $H_2O$ and sonicated in disrupt-buffer containing 1 % SDS/0.1 % beta-mercaptoethanol in PBS (pH 7.2) at 70°C for 30 minutes, followed by sonication in H2O for another 45 minutes. The T3 CLIPS carrying peptides were made in a similar way but now with three cysteines.

ELISA Screening

The binding of antibody to each of the synthesized peptides was tested in a PEPSCAN-based ELISA. The peptide arrays were incubated with primary antibody solution (overnight at 4°C). After washing, the peptide arrays were incubated with a 1/1000 dilution of an appropriate antibody peroxidase conjugate (SBA; Table 3) for one hour at 25°C. After washing, the peroxidase substrate 2,2'-azino-di-3-ethylbenzthiazoline sulfonate (ABTS) and 20 $\mu$l/ml of 3 percent $H_2O_2$ were added. After one hour, the colour development was measured. The colour development was quantified with a charge coupled device (CCD) - camera and an image processing system.

Table 3. Details of the antibodies

| Name | Supplier | Cat. No |
|---|---|---|
| goat anti-human HRP conjugate | Southern Biotech | 2010-05 |

Data Processing

The values obtained from the CCD camera range from 0 to 3000 mAU, similar to a standard 96-well plate ELISA-reader. The results are quantified and stored into the Peplab database. Occasionally a well contains an air-bubble resulting in a false-positive value, the cards are manually inspected and any values caused by an air-bubble are scored as 0.

Synthesis Quality Control

To verify the quality of the synthesized peptides, a separate set of positive and negative control peptides was synthesized in parallel. These were screened with antibody 57.9 *(ref.* Posthumus et al., J. Virology, 1990, 64:3304-3309).

Literature References

Timmerman et al. (2007). Functional reconstruction and synthetic mimicry of a conformational epitope using CLIPS™ technology. J. Mol. Recognit. 20:283-99;

Langedijk et al. (2011). Helical peptide arrays for lead identification and interaction site mapping, Analytical Biochemistry 417:149-155.

Design of Peptides

Different sets of peptides were synthesized (see also Methods section) according to the following designs. Note that actual order of peptides on mini-cards was randomized for some peptide sets described below.

**Set 1**

| Mimic Type | linear |
| --- | --- |
| Label | LIN |
| Description | Linear peptides of length 15 derived from the target sequence of human LAG-3 with an offset of one residue. |

|  |  | SEQ ID NO: |
| --- | --- | --- |
| Sequences (first 10) | VPVVWAQEGAPAQLP | 25 |
|  | PVVWAQEGAPAQLPC | 26 |
|  | VVWAQEGAPAQLPCS | 27 |
|  | VWAQEGAPAQLPCSP | 28 |
|  | WAQEGAPAQLPCSPT | 29 |
|  | AQEGAPAQLPCSPTI | 30 |
|  | QEGAPAQLPCSPTIP | 31 |
|  | EGAPAQLPCSPTIPL | 32 |
|  | GAPAQLPCSPTIPLQ | 33 |
|  | APAQLPCSPTIPLQD | 34 |

**Set 2**

| | |
|---|---|
| Mimic Type | linear |
| Label | LIN.AA |
| Description | Peptides of set 1, but residues on positions 10 and 11 replaced by Ala. When a native Ala would occur on either position, it is replaced by Gly. |

| | | SEQ ID NO: |
|---|---|---|
| Sequences (first 10) | AQRSGRAPGGAPAGH | 35 |
| | NATVTLAIIAATPKS | 36 |
| | RDGFNVSIMAALTVL | 37 |
| | IPLQDLSLLAAAGVT | 38 |
| | WGPRPRRYTAASVGP | 39 |
| | PPTPLTVYAAGGSRV | 40 |
| | SSWGPRPRRAAVLSV | 41 |
| | AGEYRAAVHAADRAL | 42 |
| | FLFLPQVSPAASGPW | 43 |
| | PGSLRASDWAALNCS | 44 |

**Set 3**

| | |
|---|---|
| Mimic Type | Looped peptides, mP2 CLIPS |
| Label | LOOP |
| Description | Constrained peptides of length 17. On positions 2-16 are 15-mer peptides derived from the target sequence of human LAG3 with an offset of one residue. On positions 1 and 17 are Cys residues that are joined by mP2 CLIPS. Native Cys residues were replaced by Cys-Acm (denoted "2"). |

| | | SEQ ID NO: |
|---|---|---|
| Sequences (first 10) | CVPVVWAQEGAPAQLPC | 45 |
| | CPVVWAQEGAPAQLP2C | 46 |
| | CVVWAQEGAPAQLP2SC | 47 |
| | CVWAQEGAPAQLP2SPC | 48 |
| | CWAQEGAPAQLP2SPTC | 49 |
| | CAQEGAPAQLP2SPTIC | 50 |
| | CQEGAPAQLP2SPTIPC | 51 |
| | CEGAPAQLP2SPTIPLC | 52 |

```
CGAPAQLP2SPTIPLQC          53
CAPAQLP2SPTIPLQDC          54
```

**Set 4**

Mimic Type    β-strand mimics, mP2 CLIPS

Label         BET

Description    Constrained peptides of length 22. On positions 2-21 are 20-mer peptides derived from the target sequence of human LAG3 with an offset of one residue. "PG" motif is inserted on positions 10 and 12 to enforce a β-turn. On positions 1 and 22 are Cys residues joined by mP2 CLIPS. Native Cys residues were replaced by Cys-Acm (denoted "2").

<u>SEQ ID NO:</u>

```
Sequences (first 10)  CVPVVWAQEGPGAQLP2SPTIC      55
                      CPVVWAQEGAPGQLP2SPTIPC      56
                      CVVWAQEGAPPGLP2SPTIPLC      57
                      CVWAQEGAPAPGP2SPTIPLQC      58
                      CWAQEGAPAQPG2SPTIPLQDC      59
                      CAQEGAPAQLPGSPTIPLQDLC      60
                      CQEGAPAQLPPGPTIPLQDLSC      61
                      CEGAPAQLP2PGTIPLQDLSLC      62
                      CGAPAQLP2SPGIPLQDLSLLC      63
                      CAPAQLP2SPPGPLQDLSLLRC      64
```

**Set 5**

| | |
|---|---|
| Mimic Type | Disulfide bridge mimics |
| Label | CYS.A |
| Description | Combinatorial mimics of length 27. On positions 1-11 and 17-27 are 11-mers that are combined via a "GGSGG" linkler based on uniprot information on disulfide bridges. Only those pairs were crated where the left and the right part of the mimic would contain pairing Cys. Cys residues not participating in the disulfide formation were replaced by Cys-Acm (denoted "2"). |

|  |  | SEQ ID NO: |
|---|---|---|
| Sequences (first 10) | AQEGAPAQLPCGGSGGAVHLRDRALSC | 65 |
| | QEGAPAQLPCSGGSGGAVHLRDRALSC | 66 |
| | EGAPAQLPCSPGGSGGAVHLRDRALSC | 67 |
| | GAPAQLPCSPTGGSGGAVHLRDRALSC | 68 |
| | APAQLPCSPTIGGSGGAVHLRDRALSC | 69 |
| | PAQLPCSPTIPGGSGGAVHLRDRALSC | 70 |
| | AQLPCSPTIPLGGSGGAVHLRDRALSC | 71 |
| | QLPCSPTIPLQGGSGGAVHLRDRALSC | 72 |
| | LPCSPTIPLQDGGSGGAVHLRDRALSC | 73 |
| | PCSPTIPLQDLGGSGGAVHLRDRALSC | 74 |

**Set 6**

| | |
|---|---|
| Mimic Type | Discontinuous mimics, T3 CLIPS |
| Label | MAT |
| Description | Combinatorial peptides of length 33. On positions 1-15 and 17-32 are 15-mer peptides derived from the target sequence of human LAG3. On positions 1, 17 and 33 are Cys residues joined by T3 CLIPS for creating a 3D structure mimic. Native Cys residues were replaced by Cys-Acm (denoted "2"). |

SEQ ID NO:

| Sequences (first 10) | | |
|---|---|---|
| | CRLGQASMTASPPGSLCFRNRGQGRVPVRESPC | 75 |
| | CVPVVWAQEGAPAQLPCWAQEGAPAQLP2SPTC | 76 |
| | CSFSRPDRPASVHWFRCESPHHHLAESFLFLPC | 77 |
| | CAQAGTYT2HIHLQEQCGDFTLRLEDVSQAQAC | 78 |
| | CLRDRALS2RLRLRLGCESPHHHLAESFLFLPC | 79 |
| | CSFLFLPQVSPMDSGPCGAPAQLP2SPTIPLQC | 80 |
| | CGTRSFLTAKWTPPGGCFLTAKWTPPGGGPDLC | 81 |
| | CTYRDGFNVSIMYNLTCVQLDERGRQRGDFSLC | 82 |
| | CLQPRVQLDERGRQRGCLQPRVQLDERGRQRGC | 83 |
| | CWAQEGAPAQLP2SPTCG2ILTYRDGFNVSIMC | 84 |

## Screening Details

Antibody binding depends on a combination of factors, including concentration of the antibody and the amounts and nature of competing proteins in the ELISA buffer. Also, the pre-coat conditions (the specific treatment of the peptide arrays prior to incubation with the experimental sample) affect binding. These details are summed up in Table 4. For the Buffer and Preconditioning (SQ), the numbers indicate the relative amount of competing protein (a combination of horse serum and ovalbumin).

Table 4. Screening conditions

| Label | Dilution | Sample buffer | Pre-conditioning |
|---|---|---|---|
| IMP761 | 9 μg/ml | 0.1% SQ | 0.1% SQ |

## Results

Primary Experimental Results and Signal to Noise Ratio Determination

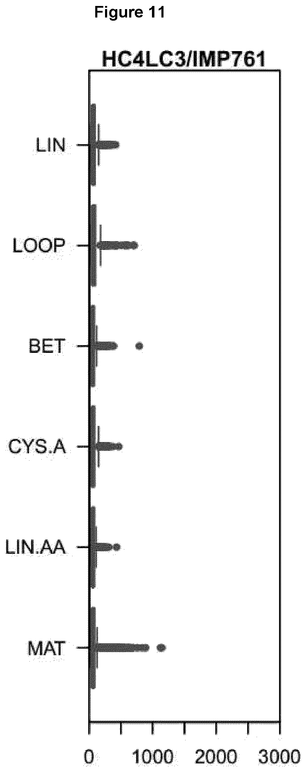

TA graphical overview of the complete dataset is given in Figure 11. A box plot depicts each dataset and indicates the average ELISA signal, the distribution and the outliers within each dataset. Depending on experimental conditions (amount of antibody, blocking strength, etc) different distributions of ELISA data were obtained.

Antibody IMP761

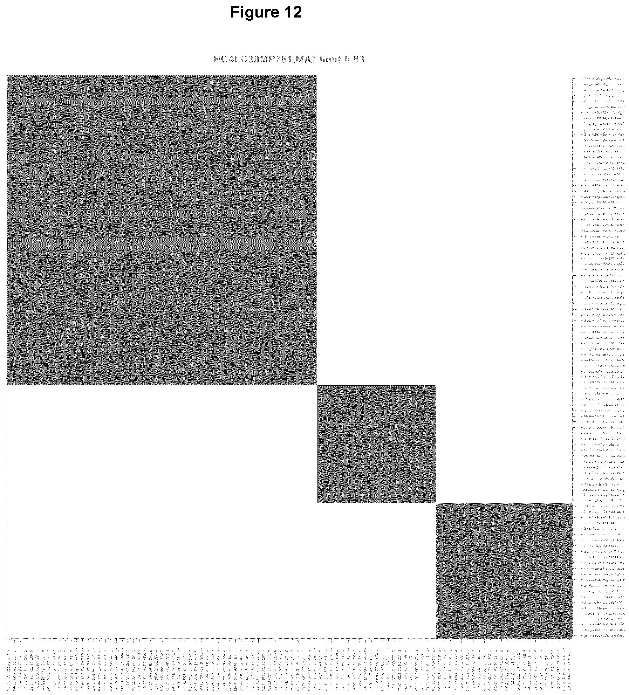

When tested under high stringency conditions antibody IMP761 did not bind any peptide present on the arrays. When tested under low stringency conditions the antibody bound peptides from all sets (Figure 12). Comprehensive data analysis suggests that IMP761 recognizes the same discontinuous epitope composed of peptide stretches $_{47}$TIPLQDLSLLRR$_{58}$ (SEQ ID NO:19), $_{107}$GGLRSGRLPLQ$_{117}$ (SEQ ID NO:20), $_{128}$QRGDFSLWLRPAR$_{140}$(SEQ ID NO:21), $_{153}$LRDRALSCRL$_{162}$ (SEQ ID NO:22).

**Conclusions**

Antibody IMP761 was tested using peptide arrays containing linear and conformational epitope mimics. Recorded data allowed proposing a discontinuous epitope candidate for IMP761 (Table 5) that is located within the Ig-like V domain D1 of human LAG-3.

Table 5. List of epitope candidates found in this study

| Antibody | Epitope candidate(s) |
|---|---|
| IMP761 | $_{47}$TIPLQDLSLLRR$_{58}$, $_{107}$GGLRSGRLPLQ$_{117}$, $_{128}$QRGDFSLWLRPAR$_{140}$, $_{153}$LRDRALSCRL$_{162}$ |

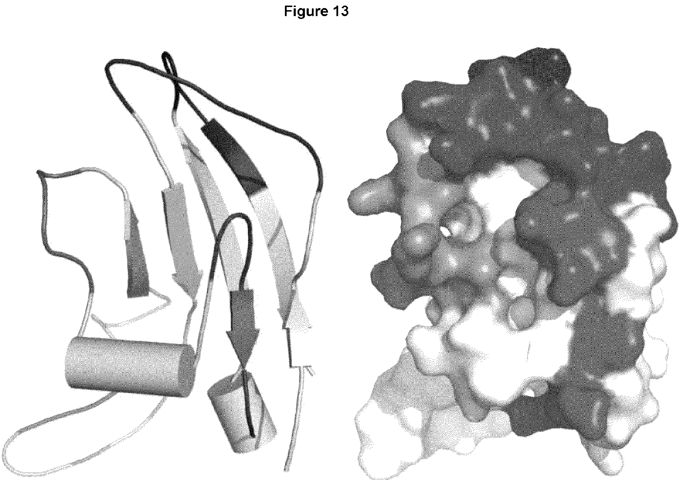

Structural evaluation of identified epitope candidates was performed using a homology model obtained for the Ig-like V domain D1 of human LAG3 (Figure 13). Despite relatively low sequence identifies between the target sequence and the template used for modelling (about 20%), identities of secondary structure elements were high (above 50%). Given the high degree of structural conservation of Ig-like domains the homology model in Figure 6 was considered moderately reliable and, therefore, included in the analysis.

Antibody IMP761 recognizes a discontinuous epitope composed of loops and secondary structure elements ($\alpha$-helical and $\beta$-strand), which are brought together by tertiary folding (Figure 13).

**Claims**

1. An isolated binding molecule, which is an agonist of LAG-3, and which inhibits TCR-mediated signal transduction in LAG-3 positive T cells through agonism of LAG-3 by inhibiting a calcineurin-NFAT signalling pathway in the LAG-3 positive T cells, wherein the binding molecule comprises an antibody, or an antigen-binding fragment thereof,

   wherein the binding molecule binds specifically to a discontinuous epitope within the extracellular Ig superfamily domain D1 (SEQ ID NO:14) of a LAG-3 protein, wherein amino acid residues of the discontinuous epitope lie outside a 30 amino acid extra-loop sequence (SEQ ID NO:18) of the domain D1 of the LAG-3 protein, and wherein the discontinuous epitope comprises the following amino acid sequences:

   TIPLQDLSLLRR (SEQ ID NO:19);
   GGLRSGRLPLQ (SEQ ID NO:20);
   QRGDFSLWLRPAR (SEQ ID NO:21); and
   LRDRALSCRL (SEQ ID NO:22); and

   wherein the binding molecule comprises:
   a variant of an antibody, or antigen-binding fragment thereof, in which no more than one, two, three, four or five amino acid residues are altered by amino acid substitution, addition, or deletion, within the CDR sequences of the antibody, or antigen-binding fragment thereof, wherein the antibody, or antigen-binding fragment thereof, of which no more than one, two, three, four or five amino acid residues are altered in the variant, comprises:
   an antibody VH region comprising:

   a VH CDR1 with an amino acid sequence selected from SEQ ID NO:1 and 7;
   a VH CDR2 with an amino acid sequence selected from SEQ ID NO:2 and 8; and
   a VH CDR3 with an amino acid sequence selected from SEQ ID NO:3 and 9; and

   an antibody VL region comprising:

   a VL CDR1 with an amino acid sequence selected from SEQ ID NO:4 and 10;
   a VL CDR2 with an amino acid sequence selected from SEQ ID NO:5 and 11; and
   a VL CDR3 with an amino acid sequence selected from SEQ ID NO:6 and 12; and
   wherein the binding molecule excludes an antibody, or antigen-binding fragment thereof, that comprises VH CDRs1-3 (SEQ ID NOs:1-3, or 7-9) and VL CDRs1-3 (SEQ ID NOs:4-6, or 10-12) of antibody IMP761.

2. A binding molecule according to claim 1, which inhibits expression of one or more NFAT-regulated genes, wherein the one or more NFAT-regulated genes include genes encoding cytokines, receptors, or nuclear factors, and/or genes encoding IL-2, IFN-y, IL-4, IL-6, IL-17, IL-21, IL-22, IL-23, CD25, PD-1, or TIM-3.

3. A binding molecule according to claim 2, wherein the one or more NFAT-regulated genes include a gene encoding IL-2.

4. A binding molecule according to any preceding claim, which inhibits antigen-induced CD4$^+$ and/or CD8$^+$ T-cell proliferation, and/or antigen-induced CD4$^+$ and/or CD8$^+$ T-cell activation.

5. A binding molecule according to any preceding claim, which binds to a human LAG-3 protein (or a human LAG-3Ig protein) with a dissociation constant ($K_D$) of no more than 100 pM, no more than 90 pM, no more than 80 pM, no more than 70 pM, no more than 60 pM, no more than 50 pM, no more than 40 pM, no more than 30 pM, or no more than 25 pM, for example as determined by Biacore analysis.

6. A binding molecule according to any preceding claim, wherein the binding molecule comprises one, two, or three complementarity determining regions (CDRs) of an antibody heavy chain variable (VH) region of IMP761 (VH CDRs1-3: SEQ ID NOs:1-3 or 7-9), and/or one, two, or three CDRs of an antibody light chain variable (VL) region of IMP761 (VL CDRs1-3: SEQ ID NOs:4-6 or 10-12), excluding an antibody, or antigen-binding fragment thereof, that comprises VH CDRs1-3 (SEQ ID NOs:1-3 or 7-9) and VL CDRs1-3 (SEQ ID NOs:4-6 or 10-12) of antibody IMP761.

7. A pharmaceutical composition comprising a binding molecule according to any preceding claim, and a pharmaceutically acceptable carrier, excipient, or diluent.

8. A binding molecule according to any of claims 1 to 6, or a pharmaceutical composition according to claim 7, for use as a medicament.

9. A binding molecule according to any of claims 1 to 6, or a pharmaceutical composition according to claim 7, for use in the treatment of a T cell-mediated immune disorder, wherein the T-cell-mediated immune disorder is an inflammatory disease, or an autoimmune disorder.

10. A binding molecule or a pharmaceutical composition for use according to claim 9, wherein the T-cell-mediated immune disorder is selected from the group consisting of infections (viral, bacterial, fungal and parasitic), endotoxic shock associated with infection, sepsis, arthritis, rheumatoid arthritis, asthma, COPD, pelvic inflammatory disease, Alzheimer's Disease, inflammatory bowel disease, Crohn's disease, ulcerative colitis, Peyronie's Disease, coeliac disease, gallbladder disease, Pilonidal disease, peritonitis, psoriasis, vasculitis, surgical adhesions, stroke, Type I Diabetes, lyme disease, arthritis, meningoencephalitis, autoimmune uveitis, immune mediated inflammatory disorders of the central and peripheral nervous system such as multiple sclerosis, lupus (such as systemic lupus erythematosus) and Guillain-Barré syndrome, Atopic dermatitis, autoimmune hepatitis, fibrosing alveolitis, Grave's disease, IgA nephropathy, idiopathic thrombocytopenic purpura, Meniere's disease, pemphigus, primary biliary cirrhosis, sarcoidosis, scleroderma, Wegener's granulomatosis, other autoimmune disorders, pancreatitis, trauma (surgery), graft-versus-host disease, transplant rejection, heart disease including ischaemic diseases such as myocardial infarction as well as atherosclerosis, intravascular coagulation, bone resorption, osteoporosis, osteoarthritis, periodontitis and hypochlorhydia, or infertility related to lack of fetal-maternal tolerance.

Figure 1

# Figure 2

## Figure 3

```
        10         20         30         40         50         60
LQPGAEVPVV WAQEGAPAQL PCSPTIPLQD LSLLRRAGVT WQHQPDSGPP AAAPGHPLAP

        70         80         90        100        110        120
GPHPAAPSSW GPRPRRYTVL SVGPGGLRSG RLPLQPRVQL DERGRQRGDF SLWLRPARRA

       130        140        150        160        170        180
DAGEYRAAVH LRDRALSCRL RLRLGQASMT ASPPGSLRAS DWVILNCSFS RPDRPASVHW

       190        200        210        220        230        240
FRNRGQGRVP VRESPHHHLA ESFLFLPQVS PMDSGPWGCI LTYRDGFNVS IMYNLTVLGL

       250        260        270        280        290        300
EPPTPLTVYA GAGSRVGLPC RLPAGVGTRS FLTAKWTPPG GGPDLLVTGD NGDFTLRLED

       310        320        330        340        350        360
VSQAQAGTYT CHIHLQEQQL NATVTLAIIT VTPKSFGSPG SLGKLLCEVT PVSGQERFVW

       370        380        390        400        410        420
SSLDTPSQRS FSGPWLEAQE AQLLSQPWQC QLYQGERLLG AAVYFTELSS PGAQRSGRAP

       430        440        450        460        470        480
GALPAGHLLL FLTLGVLSLL LLVTGAFGFH LWRRQWRPRR FSALEQGIHP QAQSKIEELE

       490        500
QEPEPEPEPE PEPEPEPEPE QL
```

Figure 4

# Figure 5

Figure 6

# Figure 7

Figure 8

Figure 9

# Figure 10

EP 4 609 916 A2

**A**

| Loop 1 | Loop 2 | ELISA |
|---|---|---|
| CMDYDFKVKLSSERERCWAIGCIFAELLTSEPC | | -0.01 |
| CMDYDFKVKLSSERERCCIFAELLTSEPIFHCC | | 0.79 |
| CMDYDFKVKLSSERERCELLTSEPIFHCRQEDC | | 1.21 |
| CMDYDFKVKLSSERERCSEPIFHCRQEDIKTSC | | 0.36 |
| CFKVKLSSERERVEDLCWAIGCIFAELLTSEPC | | 0.17 |
| CFKVKLSSERERVRDLCCIFAELLTSEPIFHCC | | 1.19 |
| CFKVKLSSERERVEDLCELLTSEPIFHCRQEDC | | 1.24 |
| CFKVKLSSERERVEDLCSEPIFHCRQEDIKTSC | | 0.56 |
| CLSSERERVEDLFEYECWAIGCIFAELLTSEPC | | 0.61 |
| CLSSERERVEDLFEYECCIFAELLTSEPIFHCC | | 1.21 |
| CLSSERERVEDLFEYECELLTSEPIFHCRQEDC | | 1.41 ← |
| CLSSERERVEDLFEYECSEPIFHCRQEDIKTSC | | 0.58 |
| CRERVEDLFEYEGCKVCWAIGCIFAELLTSEPC | | 0.10 |
| CRERVEDLFEYEGCKVCCIFAELLTSEPIFHCC | | 0.83 |
| CRERVEDLFEYEGCKVCELLTSEPIFHCRQEDC | | 1.21 |
| CRERVEDLFEYEGCKVCSEPIFHCRQEDIKTSC | | -0.20 |

**B**

Loop 2

| Loop 1 | WAIGCIFAELLTSEP | CIFAELLTSEPIFHC | ELLTSEPIFHCRQED | SEPIEHCRQEDIKTS |
|---|---|---|---|---|
| MDYDFKVKLSSERER | -0.01 | 0.79 | 1.21 | 0.36 |
| FKVKLSSERERVEDL | 0.17 | 1.19 | 1.24 | 0.56 |
| LSSERERVEDLFEYE | 0.61 | 1.21 | 1.41 | 0.58 |
| RERVEDLFEYEGCKV | 0.10 | 0.83 | 1.21 | -0.02 |

**C**

Low        Average        High

**D**

| | WAIGCIFAELLTSEP | CIFAELLTSEPIFHC | ELLTSEPIFHCRQED | SEPIEHCRQEDIKTS |
|---|---|---|---|---|
| MDYDFKVKLSSERER | -0.01 | 0.79 | 1.21 | 0.36 |
| FKVKLSSERERVEDL | 0.17 | 1.19 | 1.24 | 0.56 |
| LSSERERVEDLFEYE | 0.61 | 1.21 | 1.41 | 0.58 |
| RERVEDLFEYEGCKV | 0.10 | 0.83 | 1.21 | -0.02 |

**Figure 11**

HC4LC3/IMP761

# Figure 12

HC4LC3/IMP761,MAT limit:0.83

Figure 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2017037203 A
- US 20110150892 A1
- US 20110008331 A

### Non-patent literature cited in the description

- **LEE et al.** *Frontiers in Immunology*, 2018, vol. 9 (2747)
- **MAÇON-LEMAÎTRE et al.** *Immunology.*, June 2005, vol. 115 (2), 170-178
- **WORKMAN et al.** *J. Immunol.*, 2002, vol. 169, 5392-5395
- **LOUZALEN et al.** *Eur. J. Immunol.*, 2001, vol. 31, 2885-2891
- **MAEDA et al.** *J. Biol. Chem.*, 2019, vol. RA119, 007455
- **POIRIER et al.** *Clinical and Experimental Immunology*, 2011, vol. 164, 265-274
- **VAETH** ; **FESKE**. *F1000 Faculty Rev*, 2018, vol. 260
- **KLEIN-HESSLING et al.** *Nature Communications*, 2017, vol. 8, 511
- **ROSS** ; **CANTRELL**. *Annual Review of Immunology*, 2018, vol. 36, 411-33
- **HUARD et al.** *Proc. Natl. Acad. Sci. USA*, 1997, vol. 11, 5744-5749
- **MURPHY et al.** Using Biacore to measure the binding kinetics of an antibody-antigen interaction;. *Curr Protoc Protein Sci.*, September 2006 (19)
- **LOWRY et al.** Protein measurement with the Folin phenol reagent. *Journal of Biological Chemistry*, 1951, vol. 193 (1), 265-75
- **BRUMMELL et al.** *Biochem*, 1993, vol. 32, 1180-8
- **WILDT et al.** *Prot. Eng*, 1997, vol. 10, 835-41
- **KOMISSAROV et al.** *J. Biol. Chem*, 1997, vol. 272, 26864-26870
- **HALL et al.** *J. Immunol.*, 1992, vol. 149, 1605-12
- **KELLEY** ; **O'CONNELL**. *Biochem.*, 1993, vol. 32, 6862-35
- **ADIB-CONQUY et al.** *Int. Immunol.*, 1998, vol. 10, 341-6
- **BEERS et al.** *Clin. Can. Res*, 2000, vol. 6, 2835-43
- **CHEN**. *Drug Dev Ind Pharm*, 1992, vol. 18, 1311-54
- **REMINGTON**. Pharmaceutical Sciences. Mack Publishing Company, 1985
- **LESCZEK KROWCZYNSKI**. Extended-Release Dosage Forms. CRC Press, Inc, 1987
- **AGIS F. KYDONIEUS**. Controlled Release Technologies: Methods, Theory and Applications. CRC Press, Inc., 1980
- **YIE W. CHIEN**. Novel Drug Delivery Systems. Marcel Dekker, Inc., 1992
- **TIMMERMAN et al.** *J. Mol. Recognit.*, 2007, vol. 20, 283-29
- **POSTHUMUS et al.** *J. Virology*, 1990, vol. 64, 3304-3309
- **TIMMERMAN et al.** Functional reconstruction and synthetic mimicry of a conformational epitope using CLIPS™ technology. *J. Mol. Recognit.*, 2007, vol. 20, 283-99
- **LANGEDIJK et al.** Helical peptide arrays for lead identification and interaction site mapping. *Analytical Biochemistry*, 2011, vol. 417, 149-155